(19) Europäisches Patentamt
European Patent Office
Office européen des brevets

(11) EP 4 606 436 A2

(12) EUROPÄISCHE PATENTANMELDUNG

(43) Veröffentlichungstag:
**27.08.2025 Patentblatt 2025/35**

(21) Anmeldenummer: **25188716.2**

(22) Anmeldetag: **10.02.2021**

(51) Internationale Patentklassifikation (IPC):
***A61Q 11/00*** *(2006.01)*

(52) Gemeinsame Patentklassifikation (CPC):
**C07H 3/02; A23L 2/60; A23L 27/33; A23L 29/30; A61K 8/0241; A61K 8/60; A61Q 11/00; C07H 1/00; C07H 1/06**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.02.2020 EP 20156808**
**12.02.2020 EP 20156862**

(62) Dokumentnummer(n) der früheren Anmeldung(en) nach Art. 76 EPÜ:
**21704535.0 / 4 103 575**

(71) Anmelder: **SAVANNA Ingredients GmbH**
**50189 Elsdorf (DE)**

(72) Erfinder:
- **BACHMANN, Vanessa**
**41065 Mönchengladbach (DE)**
- **KLEIN, Carolin, Ellen**
**50389 Wesseling (DE)**
- **VEHLOW, Nathalie**
**67454 Haßloch (DE)**
- **KAUFMANN, Birgit**
**50933 Köln (DE)**
- **KOCH, Timo Johannes**
**50189 Elsdorf (DE)**
- **JUNKLEWITZ, Anna**
**42119 Wuppertal (DE)**
- **MOHR, Stephan**
**53879 Euskirchen (DE)**
- **BUTZ, Steffen**
**52393 Hürtgenwald (DE)**

(74) Vertreter: **Kutzenberger Wolff & Partner**
**Waidmarkt 11**
**50676 Köln (DE)**

Bemerkungen:
Diese Anmeldung ist am 10-07-2025 als Teilanmeldung zu der unter INID-Code 62 erwähnten Anmeldung eingereicht worden.

# (54) ALLULOSE IN KRISTALLINER FORM

(57) Vorgeschlagen wird eine kristalline Allulose mit einer definierten Partikelgrößenverteilung, ein Verfahren zu ihrer Herstellung sowie ihre Verwendung. Die neue Allulosequalität zeichnet sich dadurch aus, dass sie die Haltbarkeit damit konfektionierter Endprodukte sowie deren sensorischen und geschmacklichen Eigenschaften verbessert.



Processed by Luminess, 75001 PARIS (FR)

## Beschreibung

### GEBIET DER ERFINDUNG

Die Erfindung befindet sich auf dem Gebiet der Lebensmitteltechnologie und betrifft. eine neue kristalline Allulose, die durch eine spezielle Partikelgrößenverteilung charakterisiert ist, ein Verfahren zu ihrer Herstellung sowie ihre Verwendung zur Herstellung von oralen Zubereitungen.

### TECHNOLOGISCHER HINTERGRUND

Aufgrund des wachsenden Interesses eines großen Teils der Bevölkerung an "*gesunder Ernährung"* und "*gesunder Lebensweise"* allgemein, haben kalorienfreie oder zumindest kalorienarme Zucker und Zuckerersatzstoffe ein großes Interesse in der Lebensmittelindustrie und in der wissenschaftlichen Community erweckt.

Unter die Zuckerersatzstoffe werden die Süßstoffe und die Zuckeralkohole gerechnet. Süßstoffe können auf natürlichem oder synthetischem Weg hergestellt werden. Sie zeichnen sich dadurch aus, dass sie keinen oder nur einen vernachlässigbar geringfügigen Nährwert besitzen, was sie für Menschen, die Diät halten, besonders interessant macht.

Süßstoff scheidet der Körper entweder ganz oder größtenteils unverändert aus. Sie finden sich in vielen Light- und Diätprodukten. Zuckeraustauschstoffe sind Kohlenhydrate, die nur einen geringen Anstieg des Blutzucker- und Insulinspiegels verursachen. Ihre Süßkraft beträgt 40 bis 70 Prozent der Süßkraft des normalen Haushaltszuckers. Der Anteil von Zuckerersatzstoffen in Lebensmitteln und Getränken hat in den letzten Jahren kontinuierlich zugenommen. Im Rahmen der vorliegenden Erfindung soll unter dem Begriff "*niedrigkalorisch*" ein Brennwert von nicht mehr als 10, vorzugsweise nicht mehr als 5 und insbesondere von 1 bis 2 kcal verstanden werden.

Die industriell bedeutsamsten Zuckerersatzstoffe sind Acesulfam K und Aspartam. Es handelt sich hierbei um synthetische Stoffe, die in einigen Studien als krebsgefährdend bezeichnet werden. Diese bisher nicht bestätigten Befunde stärken jedoch den allgemeinen Trend hin zu "*natürlichen*" *Zu*ckerersatzstoffen.

Zu den wichtigsten natürlichen Zuckerersatzstoffen gehören Stevia, Erythrit, Xylit, Sucralose und Sorbit. Die Süßkraft dieser Stoffe im Vergleich zu Saccharose liegt zwischen 0,5 und 600, die Kalorienzahl zwischen 0 und 260. Alle diese Stoffe weisen jedoch Nachteile auf, zum Beispiel:

- Stevia hat einen intensiven unangenehmen metallischen Eigengeschmack und ist zum Backen ungeeignet;
- Erythrit und Xylit lassen sich weder karamellisieren noch bräunen (Maillard-Reaktion);
- Sucralose ist für Menschen mit Fructoseintoleranz ungeeignet;
- Sorbit ist für viele Menschen, insbesondere für Diabetiker unverträglich.

Aber auch bei den konfektionierten Produkten gibt es Raum für Verbesserungen, angefangen bei der zu verbessernden Haltbarkeit von Nahrungsmitteln im Allgemeinen oder speziell die mangelnde Farbbeständigkeit von Tomatenketchup, die nicht zufriedenstellende Löslichkeit vieler Instantgetränke, speziell im Automatenbereich, bis hin zu sensorischen Effekten wie der mangelnden Cremigkeit von Speiseeis, Joghurt oder Frischkäse, um nur einige exemplarischen Mängel zu nennen.

Diese Lücke könnte geschlossen werden durch die so genannten *"seltenen Zuckern"* wie Tagatose, Cellobiose oder Allulose (die synonym auch als Psicose bezeichnet wird). Hierbei handelt es sich um Einfachzucker, die zwar nicht an die Süßkraft von Haushaltszucker herankommen, vom menschlichen Körper aber nicht verstoffwechselt werden. Sie gelten zudem als *"natürlich",* da sie in der Natur zu finden sind, wenn auch nur in geringen Mengen. Sie eignen sich daher insbesondere zum Teilaustausch in zuckerhaltigen Lebensmitteln.

Allulose (Psicose) ist ein kalorienarmer Zucker mit ähnlich süßem Geschmack von Zucker. Allulose ist einer von vielen verschiedenen Zuckern, die in der Natur in sehr geringen Mengen vorkommen. Allulose wurde ursprünglich aus Weizen identifiziert und wurde seitdem in bestimmten Früchten wie Jackfrucht, Feigen und Rosinen gefunden. Allulose ist natürlich in kleinen Mengen in einer Vielzahl von süßen Lebensmitteln wie Karamellsauce, Ahornsirup und braunem Zucker enthalten. Allulose wird vom Körper absorbiert, aber nicht metabolisiert, sodass sie nahezu kalorienfrei ist.

### RELEVANTER STAND DER TECHNIK

Verschiedene Verfahren zur Herstellung von Allulose sind aus dem Stand der Technik bekannt, wie beispielweise aus den Dokumenten WO 2018/087261 A1 (Pfeifer & Langen GmbH & Co. KG) oder WO 2016/135358 A1 (Tate & Lyle Technology Limited).

WO 2018 20103 A1 (GIVAUDAN) beansprucht eine Sweetener-Mischung aus Stevia und weiteren Süßstoffen wie z.B. Psicose. Psicose steht dabei aber nicht im Vordergrund, sondern Mogroside.

WO 2014 186084 A1 (PEPSICO) beansprucht eine Getränkezusammensetzung, die als Süßstoffkomponente Erythrol, Psicose und Rebaudiosid M enthält.

AUFGABE DER ERFINDUNG

Die Haltbarkeit von Lebensmitteln wird mit Hilfe der Wasseraktivität bzw. des so genannten $a_w$-Wertes beschrieben. Er stellt ein Maß für das "*verfügbare*" oder *"aktive"* Wasser im Gegensatz zur bloßen Angabe des Wassergehalts dar. Die Bedeutung dieser Größe ergibt sich daraus, dass für die Haltbarkeit von Lebensmitteln nicht nur der reine Wassergehalt von Bedeutung ist, sondern auch, in welchem Maße das Wasser durch das Substrat gebunden ist. Die Wasseraktivität beeinflusst das Wachstum von Mikroorganismen, den Ablauf chemischer Prozesse wie Fettoxidation und nichtenzymatische Bräunung, die Aktivität von Enzymen, und die physischen Eigenschaften des Lebensmittels. Die Wasseraktivität ist definiert als Verhältnis des Wasserdampfpartialdrucks in dem Lebensmittel (p) zum Sättigungsdampfdruck von reinem Wasser ($p_0$) bei einer bestimmten Temperatur:

$$a_w = p/P_0$$

Die Wasseraktivität ist gleichbedeutend mit der (relativen) Gleichgewichtsfeuchtigkeit (RGF), das heißt der relativen Luftfeuchtigkeit, bei der das Lebensmittel (wiederum bei der gegebenen Temperatur) mit der Umgebungsluft im Gleichgewicht steht, also weder Wasser verliert noch aufnimmt. Allerdings wird die relative Luftfeuchtigkeit meistens in der Hilfsmaßeinheit Prozent angegeben, so dass man die relative Gleichgewichtsfeuchtigkeit berechnet als:

$$RGF = a_w * 100$$

Gleichzeitig entspricht also die Wasseraktivität ($a_w$) 0-1 einer relativen Luftfeuchte von 0-100 %. Die Messung der Wasseraktivität erfolgt im einfachsten Fall, indem eine Probe des Lebensmittels in einen hermetisch verschlossenen Behälter gebracht und mit einem Hygrometer die Luftfeuchtigkeit gemessen wird, die sich in dem Behälter einstellt.

Eine Absenkung des Wasserwertes - gleichbedeutend mit der Verminderung des verfügbaren Wassers - stellt damit eine dauerhafte Herausforderung für die Lebensmittelindustrie dar, um die Haltbarkeit deren Produkte zu verbessern.

Die Aufgabe der vorliegenden Erfindung hat somit darin bestanden, einen nichtkalorischen Zuckerersatzstoff in einer neuen Form zur Verfügung zu stellen, der die Haltbarkeit von Nahrungsmitteln verlängert und die geschmacklichen und sensorischen Eigenschaften von Endzubereitungen, welche Zucker bzw. Zuckerersatzstoffe enthalten können, verbessert.

BESCHREIBUNG DER ERFINDUNG

Gegenstand der vorliegenden Erfindung ist Allulose in kristalliner Form, dadurch gekennzeichnet, dass sie eine Partikelgrößenverteilung bestimmt nach der $X_{c(min)}$-Methode aufweist, für die eine, zwei oder alle drei der folgenden Auswahlregeln gelten:

| Dispergierdruck [kPa] | Minimum d10 [μm] | Minimum d50 [μm] | Minimum d90 [μm] |
|---|---|---|---|
| 5 | > 70 | > 140 | > 230 |
| 20 | > 50 | > 120 | > 220 |
| 460 | > 10 | > 40 | > 130 |

und/oder

eine Partikelgrößenverteilung bestimmt nach der $X_{Fe(max)}$-Methode aufweist, für die eine, zwei oder alle drei der folgenden Auswahlregeln gelten:

| Dispergierdruck [kPa] | Minimum d10 [μm] | Minimum d50 [μm] | Minimum d90 [μm] |
|---|---|---|---|
| 5 | > 100 | > 220 | > 340 |
| 20 | > 70 | > 200 | > 330 |

(fortgesetzt)

| Dispergierdruck [kPa] | Minimum d10 [µm] | Minimum d50 [µm] | Minimum d90 [µm] |
|---|---|---|---|
| 460 | > 8 | > 65 | > 170 |

und/oder

eine Partikelgrößenverteilung bestimmt nach der $X_{area}$-Methode aufweist, für die eine, zwei oder alle drei der folgenden Auswahlregeln gelten:

| Dispergierdruck [kPa] | Minimum d10 [µm] | Minimum d50 [µm] | Minimum d90 [µm] |
|---|---|---|---|
| 5 | > 85 | > 185 | > 265 |
| 20 | > 65 | > 180 | > 260 |
| 460 | > 10 | > 50 | > 120 |

Die besagte bevorzugte Korngrößenverteilung wird mittels dynamischer Bildanalyse (Camsizer X2 der Firma Retsch mit X-Jet Modul) ermittelt, wobei ein Dispergierdruck von 5, 20 oder 460 kPa angelegt wurde. Die Auswertung erfolgte in drei Größenmodellen, nämlich:

- $X_{c(min)}$ Partikelbreite, welche aus der schmalsten aller gemessenen Sehnen $x_c$ bestimmt wird;
- $X_{Fe(max)}$ Partikellänge, welche aus dem längsten aller gemessenen Feret-Durchmesser $x_{Fe}$ bestimmt wird.
- $X_{area}$ äquivalenter Partikeldurchmesser, welcher dem Durchmesser eines flächengleichen Kreises entspricht

Der Wert "b/l d50" gibt jeweils das Breiten/Längenverhältnis der Kristalle beim d50 wieder. Es ist besonders bevorzugt, wenn dieser Wert zwischen 0,6 und 1,0, insbesondere zwischen 0,7 und 0,9 und optimal zwischen 0,75 und 0,8 liegt.

In einer bevorzugten Ausführungsform weist die erfindungsgemäße einen Allulose in kristalliner Form einen b/l d50-Wert auf, für den gilt: und/oder

| Parameter | Dispergierdruck [kPa] | $X_{c(min)}$ |
|---|---|---|
| b/l d50 | 5 | 0,52-1,0 |
| und/oder | | |
| b/l d50 | 20 | 0,53-1,0 |
| und/oder | | |
| b/l d50 | 460 | 0,60 - 1,0 |

und/oder:

| Parameter | Dispergierdruck [kPa] | $X_{Fe(max)}$ |
|---|---|---|
| b/l d50 | 5 | 0,52-1,0 |
| und/oder | | |
| b/l d50 | 20 | 0,55-1,0 |
| und/oder | | |
| b/l d50 | 460 | 0,65-1,0 |

| Parameter | Dispergierdruck [kPa] | $X_{area}$ |
|---|---|---|
| b/l d50 | 5 | 0,52-1,0 |
| und/oder | | |

(fortgesetzt)

| Parameter | Dispergierdruck [kPa] | $X_{area}$ |
|---|---|---|
| b/l d50 | 20 | 0,55-1,0 |
| und/oder | | |
| b/l d50 | 460 | 0,65-1,0 |

Überrascherderweise wurde gefunden, dass Allulose als Süßungsmittel den Wasserwert von konfektionierten Produkten senken und damit deren Haltbarkeit deutlich verbessern kann, wenn diese eine definierte Partikelgrößenverteilung wie oben beschrieben aufweist. Dies ist insbesondere deshalb überraschend und nicht zu erwarten gewesen, da ein Zusammenhang zwischen Durchmesser und Form von Kristallpartikeln und Wasserwert bisher nicht bekannt war. Gleichzeitig ist diese neue kristalline Allulosequalität in der Lage, die sensorischen und geschmacklichen Eigenschaften von oralen Zubereitungen in Summe und von Lebensmitteln im Besonderen in vielfacher Weise positiv zu beeinflussen, wie zum Beispiel:

- Verbesserung des Mundgefühls,
- Intensivierung und Stabilisierung der Farbe, z.B. in Orangenlimonade;
- Verstärkung der Geschmacksintensität, z.B. intensivere Fruchtigkeit der Tomate in Ketchup oder der Umaminote in Snackartiklen;
- Stabilisierung von Lebensmittelemulsionen, oder
- schnellere Löslichkeit von Instantprodukten

um nur einige zu nennen.

Wie bereits erwähnt, ist Allulose (Psicose) ist ein kalorienarmer Zucker mit einem ähnlichen süßen Geschmack von Zucker. Allulose ist einer von vielen verschiedenen Zuckern, die in der Natur in sehr geringen Mengen vorkommen. Allulose wurde erstmals in Weizen identifiziert und seitdem in bestimmten Früchten wie Jackfrucht, Feigen und Rosinen gefunden. Allulose ist natürlich in kleinen Mengen in einer Vielzahl von süßen Lebensmitteln wie Karamellsauce, Ahornsirup und braunem Zucker enthalten. Allulose wird vom Körper absorbiert, aber nicht metabolisiert, sodass sie nahezu kalorienfrei ist.

Die Herstellung von Allulose bzw. Psicose aus Fructose ist seit 1995 bekannt, wie die Auswahl der folgenden Schriften zeigt:

- H. Itoh et al, Journal of Fermentation Bioengeneering, 80(1), 1995, S. 101-103 veröffentlicht die Herstellung von D-Psicose aus D-Fructose durch immobilisierte D-Tagatose 3-Epimerase.
- N. Wagner et al in Organic Process Research Development 2012, 16, S. 323-330 bezieht sich auf praktische Aspekte des integrierten Betriebs von Biotransformation und simulierter Bewegungsbetttrennung (SMB) für die feinchemische Synthese. D-Psicose wird unter Verwendung der D-Tagatose Epimerase-katalysierten Epimerisierung aus D-Fructose hergestellt.
- N. Wagner et al, in Chemical Engineering Science 137 (2015) S. 423-435 bezieht sich auf die modellbasierte Kostenoptimierung eines integrierten Prozesses zur enzymatischen Herstellung von Psicose bei erhöhten Temperaturen.
- N. Wagner et al, in Angewandte Chemie Int. Ed. Engl. 2015, 54(14), S. 4182-6 offenbart eine trennungsintegrierte Kaskadenreaktion zur Überwindung thermodynamischer Einschränkungen bei der Seltenzucker-Synthese.
- Bosshart et al, in Biotechnology Bioengineering 2016, 113(2), S. 349-58 bezieht sich auf die Produktion der seltenen Zucker D-Psicose und L-Tagatose durch zwei künstlich hergestellte D-Tagatose-Epimerasen.
- N. Wagner, et al., in Journal of Chromatography A 2015, 1398, S. 47-56 offenbart ein Verfahren zur wirtschaftlichen Herstellung von d-Psicose durch simulierte Wanderbettchromatographie.

- Gegenstand der WO 2018 087261 A1 (PFEIFER&LANGEN) ist ein Verfahren zur Synthese eines Einfachzuckers, vorzugsweise von D-Allulose aus einem Edukt, vorzugsweise von D-Fructose unter heterogener oder homogener Katalyse, die eine chemische und/oder enzymatische Katalyse beinhaltet, wobei die Synthese in mindestens zwei in Reihe geschalteten Reaktoren durchgeführt wird und das aus dem ersten Reaktor austretende Reaktionsprodukt vor dem Eintritt in den zweiten Reaktor einer chromatographischen Trennung unterzogen wird. Vorzugsweise wird die chromatographische Trennung in ein simuliertes Wanderbett integriert.

In der Regel wird Allulose in Form eines Sirups kommerzialisiert, der nach einem der oben genannten Verfahren erhältlich ist.

Ein weiterer Gegenstand der vorliegenden Erfindung betrifft ein Verfahren zur Herstellung von kristalliner Allulose wie vorstehend beschrieben, umfassend oder bestehend aus folgenden Schritten:

(a) Bereitstellen einer wässrigen Allulose-Zubereitung, vorzugsweise einem Verdampferkonzentrat ("Allulose-Sirup"), umfassend oder bestehend aus folgenden Komponenten, bezogen auf die in der Zubereitung enthaltene Trockenmasse:

(i) etwa 90 bis etwa 99 Gew.-% Allulose,

(ii) etwa 0,5 bis etwa 10 Gew.-% Fructose,

(iii) 0 bis etwa 5 Gew.-% weitere Kohlenhydrate, verschieden von Allulose und Fructose,

mit der Maßgabe, dass sich die Mengenangaben zu 100 Gew.-% ergänzen;

(b) optionale Behandlung der Allulose-Zubereitung des Schrittes (a) mit einem Entfärbungsmittel;

(c) Aufkonzentrieren der Zubereitung aus Schritt (a) oder Schritt (b) bis die Sättigungskonzentration überschritten wird;

(d) Versetzen der übersättigten Zubereitung aus Schritt (c) mit Allulose-Impfkristallen; und

(e) Abkühlen der Zubereitung aus Schritt (c) mit einer Geschwindigkeit von 0,1 bis etwa 1 K/h bis ein Kristallgehalt von etwa 30 bis etwa 50 Gew.-% erreicht wird, sowie

(f) Abtrennen und optional Trocknen der Kristalle.

Der guten Ordnung halber sei darauf hingewiesen, dass Ausführungsformen, die sich zu mehr oder zu weniger als 100 Gew.-% ergeben, weder von der Erfindung eingeschlossen noch von den Ansprüchen umfasst werden. Der Fachmann ist in jedem Fall in der Lage, nach Maßgabe von Beschreibung und Beispielen solche Zusammensetzungen aufzufinden, die die technische Lehre erfüllen, ohne dazu erfinderisch tätig werden zu müssen.

In einer bevorzugten Ausführungsform des erfindungsgemäßen Verfahrens weist die Zubereitung des Schrittes (a) einen Gehalt an Allulose ≥ 90 Gew.-% auf, vorzugsweise ≥ 91 Gew.-%, vorzugsweise ≥ 92 Gew.-%, vorzugsweise ≥ 93 Gew.-%, vorzugsweise ≥ 94 Gew.-%, vorzugsweise ≥ 95 Gew.-%, vorzugsweise ≥ 96 Gew.-%, vorzugsweise ≥ 97 Gew.-%, vorzugsweise ≥ 98 Gew.-%, jeweils bezogen auf die in der Zubereitung enthaltene Trockenmasse.

In einer weitere Ausführungsform des erfindungsgemäßen Verfahrens weist die Zubereitung des Schrittes (a) einen Gehalt an Allulose ≤ 99 Gew.-% auf, vorzugsweise ≤ 98 Gew.-%, vorzugsweise ≤ 97 Gew.-%, vorzugsweise ≤ 96 Gew.-%, vorzugsweise ≤ 95 Gew.-%, vorzugsweise ≤ 94 Gew.-%, vorzugsweise ≤ 93 Gew.-%, vorzugsweise ≤ 92 Gew.-%, vorzugsweise ≤ 91 Gew.-%, jeweils bezogen auf die in der Zubereitung enthaltene Trockenmasse.

Wie bereits erwähnt, weist die Zubereitung des Schrittes (a) einen Gehalt an Fructose von etwa 0,5 bis etwa 10 Gew.-% Fructose, bezogen auf die in der Zubereitung enthaltene Trockenmasse, auf.

In einer bevorzugten Ausführungsform des erfindungsgemäßen Verfahrens weist die Zubereitung des Schrittes (a) einen Gehalt an Fructose von etwa 1 bis etwa 10 Gew.-% Fructose auf, bezogen auf die in der Zubereitung enthaltene Trockenmasse, bevorzugt von etwa 1 bis etwa 3 Gew.-%, und insbesondere bevorzugt von etwa 1,5 bis etwa 2,5 Gew.-%.

In einer weiteren Ausführungsform des erfindungsgemäßen Verfahrens weist die Zubereitung des Schrittes (a) einen Gehalt an Fructose von etwa 0,5 bis etwa 1 Gew.-% Fructose auf, oder von etwa 1 bis etwa 1,5 Gew.-%, oder von etwa 1,5 bis etwa 2,0 Gew.-%, oder von etwa 2,0 bis etwa 2,5 Gew.-%, oder von etwa 2,5 bis etwa 3,0 Gew.-%, oder von etwa 3,0 bis etwa 3,5 Gew.-%, oder von etwa 3,5 bis etwa 4,0 Gew.-%, oder von etwa 4,0 bis etwa 4,5 Gew.-%, oder von etwa 4,5 bis etwa 5,0 Gew.-%, oder von etwa 5,0 bis etwa 5,5 Gew.-%, oder von etwa 5,5 bis etwa 6,0 Gew.-%, oder von

etwa 6,0 bis etwa 6,5 Gew.-%, oder von etwa 6,5 bis etwa 7,0 Gew.-%, oder von etwa 7,0 bis etwa 7,5 Gew.-%, oder von etwa 7,5 bis etwa 8,0 Gew.-%, oder von etwa 8,0 bis etwa 8,5 Gew.-%, oder von etwa 8,5 bis etwa 9,0 Gew.-%, oder von etwa 9,5 bis etwa 10 Gew.-%, jeweils bezogen auf die in der Zubereitung enthaltene Trockenmasse.

Die Zubereitungen des Schrittes (a) weisen erfindungsgemäß einen Feststoffgehalt von 55 bis 98 Gew.-% und vorzugsweise von etwa 75 bis etwa 95 Gew.-% auf.

In einer weiteren Ausführungsform des erfindungsgemäßen Verfahrens weist die Zubereitung des Schrittes (a) einen Feststoffgehalt von etwa 75 Gew.-%, oder von etwa 76 Gew.-%, oder von etwa 77 Gew.-%, oder von etwa 78 Gew.-%, oder von etwa 79 Gew.-%, oder von etwa 75 Gew.-%, oder von etwa 76 Gew.-%, oder von etwa 77 Gew.-%, oder von etwa 78 Gew.-%, oder von etwa 79 Gew.-%, oder von etwa 80 Gew.-%, oder von etwa 81 Gew.-%, oder von etwa 82 Gew.-%, oder von etwa 83 Gew.-%, oder von etwa 84 Gew.-%, oder von etwa 85 Gew.-%, oder von etwa 86 Gew.-%, oder von etwa 87 Gew.-%, oder von etwa 88 Gew.-%, oder von etwa 89 Gew.-%, oder von etwa 90 Gew.-%, oder von etwa 91 Gew.-%, oder von etwa 92 Gew.-%, oder von etwa 93 Gew.-%, oder von etwa 94 Gew.-%, oder von etwa 95 Gew.-%, auf.

In einer weiteren Ausführungsform des erfindungsgemäßen Verfahrens weist die Zubereitung des Schrittes (a) einen Feststoffgehalt von etwa 75 bis etwa 80 Gew.-%, oder von etwa 80 bis etwa 85 Gew.-%, von etwa 85 bis etwa 90 Gew.-%, von etwa 90 bis etwa 95 Gew.-%, auf.

Optional können die Zubereitungen des Schrittes (a) mit einem Entfärbungsmittel, beispielsweise Aktivkohle oder Entfärberharze, behandelt werden (Schritt (b) des Verfahrens). Die Entfärberharze, die im Sinne der vorliegenden Anmeldung in Frage kommen, sind kommerziell erhältlich und für den Fachmann bestens bekannt und brauchen daher nicht näher erläutert werden.

Im Schritt (c) werden die Zubereitungen des Schrittes (a) oder die entfärbten Zubereitungen des Schrittes (a) aufkonzentriert, bis die Sättigungskonzentration überschritten wird, und zwar durch einen thermischen Verdampfungsschritt.

Im Sinne der vorliegenden Erfindung kommen verschiedene Verdampfer für die Durchführung des Konzentrierungsschrittes (c) in Frage, beispielsweise Dampfkessel, Dünnschichtverdampfer, Fallfilmverdampfer, Kesselverdampfer, Koaxialverdampfer, Naturumlaufverdampfer, Plattenverdampfer oder Zwangsumlaufverdampfer. Diese Einrichtungen sind für den Fachmann bestens bekannt und brauchen daher nicht näher erläutert werden. Der Konzentrierungsschritt findet typischerweise entweder in einem oder in zwei bis drei in Reihe geschalteten Verdampfern statt; vorzugsweise werden dabei Fallfilmverdampfer oder Plattenverdampfer verwendet.

Die Aufkonzentrierung wird in der Regel bei einer Temperatur von etwa 50 bis etwa 75 °C durchgeführt. Wird der Verfahrensschritt nicht einstufig, sondern in zwei oder drei Stufen durchgeführt, hat es sich aus energetischen Gründen als vorteilhaft erwiesen, bei der Temperaturführung den Bereich zwischen 59 und 71 °C auszulassen, also in den Verdampfern in Temperaturbereichen darüber und darunter zu arbeiten.

Im Schritt (d) wird das im Schritt (c) gewonnene Zwischenprodukt mit Impfkristallen versetzt, wobei etwa 1 bis etwa 3 und typisch 1,5 bis 2 Gew.-% Allulosekristalle - bezogen auf die Konzentrate - zum Einsatz kommen.

Die Übersättigung wird anschließend durch Verdampfung, vorzugsweise aber langsames Abkühlen (0,1-1K/h) aufrechterhalten bis sich ein Kristallgehalt von etwa 30 bis etwa 50 Gew.-% einstellt. Die Kristalle werden dann abgetrennt und optional noch getrocknet.

Ein weiterer Gegenstand der vorliegenden Anmeldung ist selbstverständlich auch Allulose in kristalliner Form (wie in den Absätzen [0017]-[0020] definiert), erhältlich durch das in den Absätzen [0025]-[0040] beschriebenen Verfahren.

Ein weiterer Gegenstand der Erfindung betrifft die Verwendung von kristalliner Allulose wie in den Absätzen [0017]-[0020] beschrieben, oder erhältlich durch das in den Absätzen [0025]-[0040] beschriebenen Verfahren zur Herstellung von oralen Zubereitungen.

Überraschenderweise wurde gefunden, dass der Austausch von Haushaltszucker gegen Allulosekristalle bei der Herstellung von oralen Zubereitungen, einen positiven Einfluss sowohl auf die sensorischen als auch physikalischen und geschmacklichen Eigenschaften der oralen Zubereitungen hat, die im Folgenden näher beschrieben werden.

Insbesondere wurde gefunden, dass beim Austausch von Haushaltszucker gegen Allulose der $a_w$-Wert der konfektionierten Produkte gesenkt wird.

In einer bevorzugten Ausführungsform handelt sich bei der oben genannten oralen Zubereitung um orale Zubereitungen, die ausgewählt sind aus der Gruppe, die gebildet wird von Nahrungsmitteln, Mund- und Zahnpflegemitteln sowie pharmazeutischen Zubereitungen.

## NAHRUNGSMITTEL

Wie bereits erwähnt, kann die kristalline Allulose wie in den Absätzen [0017]-[0020] beschrieben, oder erhältlich durch das in den Absätzen [0025]-[0040] beschriebenen Verfahren zur Herstellung von Nahrungsmittel verwendet werden. In Betracht kommen dabei beispielsweise und nicht abschließend:

- Getränke,
- Backwaren,
- Getreideprodukte,
- Knabberartikel,
- Süßwaren,
- Milchprodukte,
- Fruchtzubereitungen,
- Würzmittel,
- Gemüsezubereitungen,
- Fleischwaren oder
- Gewürze und Gewürzmischungen.

Die Gruppe der Getränke umfasst sowohl alkoholische als auch nicht-alkoholische Produkte, wie beispielsweise Kaffee, Tee, Eistee, Wein, weinhaltige Getränke, Bier, bierhaltige Getränke, Liköre, Schnäpse, Weinbrände, (karbonisierte) fruchthaltige Limonaden, (karbonisierte) isotonische Getränke und mit Geschmacksstoffen angereichertes Mineralwasser, (karbonisierte) Erfrischungsgetränke, Nektare, Schorlen, Obst- und Gemüsesäfte, Frucht- oder Gemüsesaftzubereitungen, Instantgetränke, wie beispielsweise Instant-Kakao-Getränke, Instant-Tee-Getränke, Instant-Kaffeegetränke oder Instant-Fruchtgetränke. Erfindungsgemäße Getränke mit kristalliner Allulose weisen beispielsweise die folgenden Vorteile auf:

- verbesserte Fruchtigkeit;
- keine negative Beeinflussung der Schaumstabilität;
- verbessertes Mundgefühl im Gegensatz zu anderen kalorienreduzierten Getränken;
- Süßung ohne Kalorien und ohne E-Nummer;
- echter Zucker ohne Kalorien + ohne "Off-taste";
- 100% Saccharose-Austausch technisch möglich;
- geeignet für Diabetiker;
- geeignet für sämtliche Formate (von Instant bis Biermisch);
- transparente Farbe im Endprodukt;
- hervorragende Synergien in Kombination mit Saccharose (Sensorik, Haptik, Optik);
- Saccharose-ähnliches Süßeprofil;
- erhöhte Farbintensität, z.B. bei Orangenlimonade;
- verbesserte Farbstabilität;
- verbessertes $CO_2$-Haltevermögen nach dem Öffnen der Flasche;
- verbesserte Spritzigkeit und Frische;
- verbessertes Mundgefühls; sowie
- durch schnellere Löslichkeit sehr gute Eignung für Automatengetränke (Instant).

Ebenfalls geeignet sind Backwaren, Getreideprodukte und Knabberartikel. Hierzu zählen z.B. Brot, Trockenkekse, Kuchen, Muffins, Donuts und sonstiges Gebäck. Als Knabberartikel kommen beispielsweise gebackene oder frittierte Kartoffelchips oder Kartoffelteigprodukte, Extrudate auf Mais- oder Erdnussbasis in Frage. Als Getreideprodukte sind beispielsweise Frühstückscerealien, Müsliriegel, vorgegarte Fertigreis-Produkte zu nennen.

Als Süßwaren kommen beispielsweise Schokoladen, Schokoladenriegelprodukte, sonstige Riegelprodukte, Fruchtgummi, Gummibonbons, Hart- und Weichkaramellen, Kaugummi, Tabletten, Mints, Überzüge (Fondants), Konfitüren, Fruchteis, Fruchtsoßen sowie Fruchtfüllungen in Betracht. Die Produkte aus diesen Gruppen, weisen mindestens einen der folgenden Vorteile auf:

- Süßung ohne Kalorien und ohne E-Nummer
- echter Zucker ohne Kalorien + ohne "Off-taste"
- geeignet als Feuchthaltemittel; verhindert Austrocknen, verlängert das Mindesthaltbarkeitsdatum;
- macht Produkt geschmeidiger/weicher, trocknet Produkt nicht aus wie andere Zuckerersatzstoffe
- verbessert Aroma und Fruchtigkeit von Zuckersäurecoatings (Bsp. Fuchtgummis)
- verbesserte Löslichkeit bei Komprimaten und Brausetabletten
- geeignet in Riegeln (Müsli, Proteinriegel)
- verbessertes sensorisches Profil durch Karamellnote;
- weichere und saftigere Krume im Vergleich zu anderen Zuckern;
- verbesserte Viskosität des Teiges;
- 100% Saccharose-Austausch möglich;

- geeignet für Diabetiker;
- verringerte Backzeit durch erhöhte Reaktivität der Allulose-Kristalle;
- optimales Bräunungsergebnis bei verringerten Backtemperaturen, weniger oder gar kein Zusatz von Farbstoffen erforderlich;
- bessere Verarbeitungsfähigkeiten von Backmischungen;
- Verstärkung des Umami-Geschmacks bei Snacks;
- Verstärkung der Karamellnote bei Toffees sowie
- Verstärkung des Schokoladengeschmacks und/oder der Kakaonote bei Schokoladen sowie schoko-oder kakaohaltigen Produkten.

Als Milchprodukte kommen beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolysierte Milchprotein-haltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke in Frage. Die Produkte aus diesen Gruppen, weisen mindestens einen der folgenden Vorteile auf:

- Süßung ohne Kalorien und ohne E-Nummer;
- verstärkte Süßewahrnehmung in aromatisierten Milchgetränken durch Kombination von kristalliner Allulose und Saccharose (Maillard-Reaktion) in Einsatzmengen von 2-15 Gew.-%;
- positive Geschmacksverstärkung in Gegenwart von Proteinen;
- echter Zucker ohne Kalorien + ohne "Off-taste";
- 100% Saccharose-Austausch technisch möglich;
- geeignet für Diabetiker;
- verbessertes Geschmacksprofil (z.B. Karamelleis: Kein oder verringerter Aromazusatz);
- Senkung des Gefrierpunktes und verbessertes Schmelzverhalten bei Speiseeis;
- weichere, cremigere Textur bei Joghurt und Quarkzubereitungen, Desserts und Cream-Cheese;
- erhöhtes sensorisches Kälteempfinden;
- intensivierte Fruchtigkeit in Sorbets; sowie
- Verringerung des negativen Kochgeschmack bei gängigen Erhitzungsverfahren.

Bei den Fruchtzubereitungen sind beispielsweise Konfitüren, Fruchteis, Fruchtsoßen, Fruchtfüllungen zu nennen. Auch Honig und honigartige Produkte, wie Ahornsirup werden im Sinne der Erfindung hierunter verstanden. Die Produkte aus diesen Gruppen, weisen mindestens einen der folgenden Vorteile auf:

- Süßung ohne Kalorien und ohne E-Nummer;
- echter Zucker ohne Kalorien + ohne "Off-taste";
- 100% Saccharoseaustausch sowie beliebige Kombinationen möglich;
- geeignet für Diabetiker;
- verbessertes Geschmacksprofil;
- intensivere Farbgebung;
- verlängerte Haltbarkeit durch niedrigeren aw-Wert
- Verstärkung der Geschmacksintensität (z.B. intensivere Fruchtigkeit);
- bessere Streichfähigkeit bei Fruchtaufstrichen;
- bessere Cremigkeit bei Honig und honigartigen Produkten;
- bessere Einarbeitung von Fruchtzubereitungen in Joghurt und Quarkzubereitungen, Desserts;
- gleichbleibende Fruchtigkeit bei geringerem Säurezusatz;
- geringere Schaumbildung; sowie
- Herstellung von Gelierzucker ohne Trocknung möglich.

Als Würzmittel und Gemüsezubereitungen eignen sich beispielsweise Ketchup (speziell Gewürz-, Curry- und BBQ-Ketchup), Soßen, Trockengemüse, Tiefkühlgemüse, vorgegarte Gemüse, eingekochte Gemüse. Hierzu zählen auch Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzen (englisch: Seasonings), die beispielsweise im Snackbereich Anwendung finden. Die Produkte aus diesen Gruppen, weisen mindestens einen der folgenden Vorteile auf:

- Süßung ohne Kalorien und ohne E-Nummer;
- echter Zucker ohne Kalorien und ohne "Off-taste";
- 100% Saccharose-Austausch möglich;
- geeignet für Diabetiker;
- verbesserte Farbgebung;
- verlängerte Haltbarkeit;
- Verstärkung der Geschmacksintensität, z.B. intensivere Fruchtigkeit der Tomate in Ketchup);
- verbesserte Viskosität von Ketchup; sowie
- Verstärkung des Umamigeschmacks.
- Gute stabilisierende Eigenschaften in Lebensmittelemulsionen

Zu den geeigneten Fleischwaren gehören beispielsweise Schinken, Frischwurst- oder Rohwurstzubereitungen, gewürzte oder marinierte Frisch- oder Pökelfleischprodukte.

Ebenfalls umfasst die Gruppe Gewürze und Gewürzmischungen sowie Süßkaftverstärkern, Geschmacksverstärker und Maskierungsmittel, gegebenenfalls in verkapselter Form.

Süßstoffe, deren Süßkraft verstärkt und/oder deren unangenehmen Geschmacksnoten (adstringierend, metallisch, teerig, fettig) überdeckt oder maskiert werden, umfassen beispielsweise: Sucrose/Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glycerinaldehyd, oder Maltodextrin. Ebenfalls geeignet sind pflanzliche Zubereitungen, die diese Stoffe enthalten, beispielsweise auf Basis von Zuckerrüben (Beta vulgaris ssp., Zuckerfraktionen, Zuckersirup, Melasse), Zuckerrohr (Saccharum officinarum ssp., Melasse, Zuckerrohrsirup), Ahornsirup (Acer ssp.) oder Agaven (Agavendicksaft).

In Betracht kommen auch synthetische, d.h. in der Regel enzymatisch hergestellte Stärke oder Zuckerhydrolysate (Invertzucker, Fructosesirup);

- Fruchtkonzentrate (z.B. auf Basis von Äpfeln oder Birnen);
- Zuckeralkohole (z.B. Erythritol, Threitol, Arabitol, Ribotol, Xylitol, Sorbitol, Mannitol, Dulcitol, Lactitol);
- Proteine (z.B. Miraculin, Monellin, Thaumatin, Curculin, Brazzein);
- Süßstoffe (z.B. Magap, Natriumcyclamat, Acesulfam K, Neohesperidin Dihydrochalcon, Saccharin Natriumsalz, Aspartam, Superaspartam, Neotam, Alitam, Sucralose, Stevioside, Rebaudioside, Lugduname, Carrelame, Sucrononate, Sucrooctate, Monatin, Phenylodulcin);
- Süß schmeckende Aminosäuren (z.B. Glycin, D-Leucin, D-Threonin, D-Asparagin, D-Phenylalanin, D-tryptophn, L-Prolin);
- Weitere süß schmeckende niedermolekulare Substanzen, wie z.B. Hernandulcin, Dihydrochalconglykoside, Glycyrrhizin, Glycerrhetinsäure, ihre Derivate und Salze, Extrakte von Lakritz (Glycyrrhizza glabra ssp.), Lippia dulcis Extrakte, Momordica ssp. Extrakte oder
- Einzelsubstanzen wie z.B. Momordica grosvenori [Luo Han Guo] und die daraus gewonnenen Mogroside, Hydrangea dulcis oder Stevia ssp. (z.B. Stevia rebaudiana) Extrakte, wie sie nachfolgend noch näher erläutert werden.

Rebaudioside gehören zu den Steviosiden, den Hauptbestandteilen der Pflanze *Stevia rebaudiana,* die auch als Süßkraut oder Honigkraut bezeichnet wird.

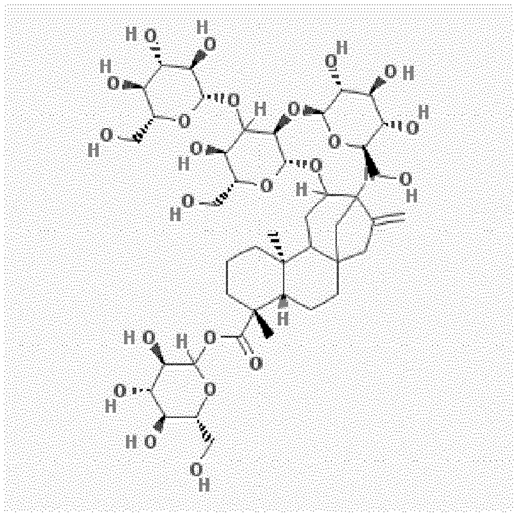

Rebaudiosid

10 % der Trockenmasse der Blätter macht das Diterpenglykoid Steviosid aus, gefolgt von Rebaudiosid A (2 bis 4 Gew.-%) sowie zehn weiteren Steviolglycosiden, wie etwa dem Dulcosid. Rebaudioside und Stevia-Extrakt sind als Süßungsmitteln inzwischen in den meisten Staaten zugelassen; eine tägliche Aufnahmemenge von bis zu 4 mg Steviosid pro Kilogramm Körpergewicht wird als unbedenklich angesehen. Im Sinne der Erfindung können einzelne Rebaudioside oder die Extrakte der Stevia-Pflanze eingesetzt werden. Besonders bevorzugt ist jedoch die Verwendung von Rebaudiosid A, da dieser Stoff eine geringere Bitterkeit und die höchste Süßkraft aufweist.

Alternativ oder ergänzend zu den Rebaudiosiden bzw. Stevia Extrakten können als Süßstoffe auch verschiedene andere Wirkstoffe eingesetzt werden; es sind dies:

- Naringin,
- Dihydrochalcone,
- Mogroside sowie
- Extrakten der Pflanzen der Gattung *Rubus.*

Naringin ist ein polyphenolisches Glykosid, das in der Grapefruit und dem Pomelo enthalten ist und diesen einen bitteren Geschmack verleiht. Der Stoff ist insbesondere wegen seiner lipidsenkenden Wirkung bekannt.

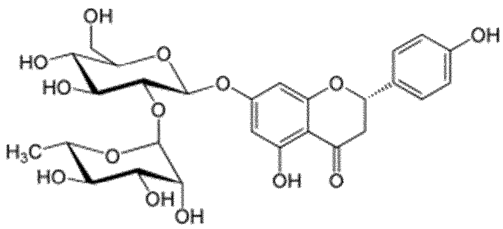

Naringin

Auch die Dihydrochalcone stellen Polyphenole dar, wobei insbesondere die beiden Vertreter Dihydrochalcon und Neohesperidin Dihydrochalcon hervorzuheben sind, die als künstliche Süßungsmittel bekannt sind:

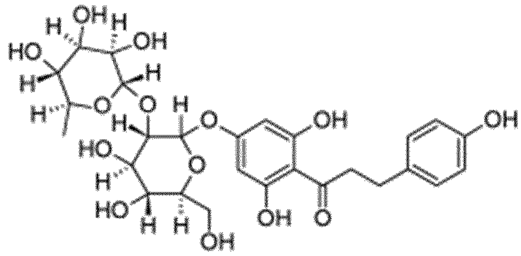

Dihydrochalcon

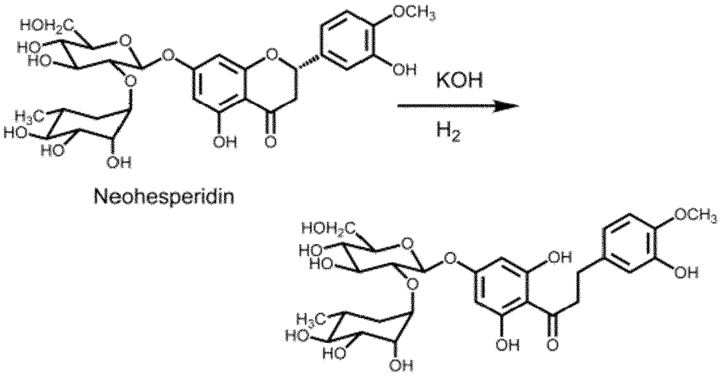


Neohesperidin

Neohesperidin dihydrochalcone

Unter der Bezeichnung Mogroside wird eine Gruppe von Glykosiden des Cucurbitans verstanden, die als Bestandteil des natürlichen Süßungsmittels Luo Han Guo bekannt sind. Hervorzuheben ist hier das Mogrosid-5, das 400mal süßer als Zucker ist.

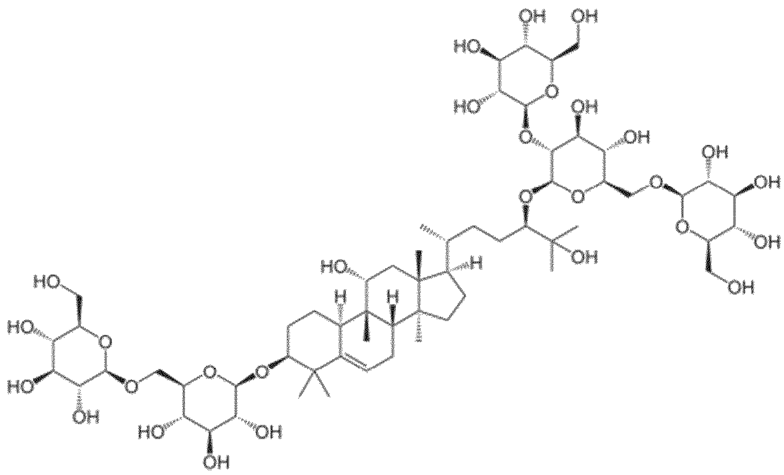

Mogrosid-5

Schließlich auch Extrakte der Pflanzen in Betracht, die ausgewählt sind aus der Gruppe, die gebildet wird von Rubus allegheniensis, Rubus arcticu, Rubus strigosus, Rubus armeniacus, Rubus caesius, Rubus chamaemorus, Rubus corylifolius agg., Rubus fruticosus agg., Rubus geoides, Rubus glaucus, Rubus gunnianus, Rubus idaeus, Rubus illecebrosus, Rubus laciniatus, Rubus leucodermis, Rubus loganobaccus, Rubus loxensis, Rubus nepalensis, Rubus nessensis, Rubus nivalis, Rubus odoratus, Rubus pentalobus, Rubus phoenicolasius, Rubus saxatilis, Rubus setchuenensis, Rubus spectabilis und Rubus ulmifolius sowie deren Gemischen. Hierbei handelt es sich im Wesentlichen um Extrakte von unterschiedlichen Brombeer- und Himbeerarten, die einen Gehalt an Rubosiden aufweisen. Bevorzugt sind Extrakte von Rubus Suavissimus.

Ein weiterer Wirkstoff in dieser Gruppe ist die Glycyrrhizinsäure oder ein entsprechendes Salz oder einen

Extrakt, der diesen Stoff enthält.

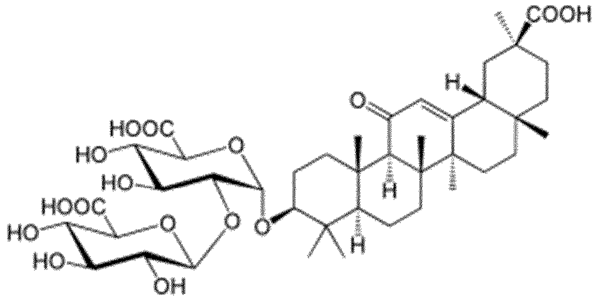

Glycyrrhizinsäure

Im Sinne der Erfindung ist es möglich, die Säure selbst, ihre Salze - beispielsweise Natrium-, Kalium- oder Ammoniumsalz - oder die Extrakte der Pflanze *Glycyrrhiza glabra* einzusetzen. Besonders bevorzugt ist das Monoammoniumglycyrrhizat.

Demzufolge ist ein weiterer Gegenstand der vorliegenden Erfindung die Verwendung von Allulose, insbesondere von kristalliner Allulose wie in den Absätzen [0017]-[0020] beschrieben, oder erhältlich durch das in den Absätzen [0025]-[0040] beschriebenen Verfahren, zur Verstärkung der Süßkraft und/oder Überdeckung und/oder Maskierung der unangenehmen Geschmacksnoten (adstringierend, metallisch, teerig, fettig) von Süßstoffen, insbesondere der oben genannten Süßstoffe.

In einer besonders bevorzugten Ausführungsform wird Allulose, insbesondere von kristalliner Allulose wie in den Absätzen [0017]-[0020] beschrieben, oder erhältlich durch das in den Absätzen [0025]-[0040] beschriebenen Verfahren, zur Maskierung der unangenehmen Geschmacksnoten (adstringierend, metallisch, teerig, fettig) von Süßstoffen, insbesondere der oben genannten Süßstoffen, verwendet. Bei einer solchen Verwendung liegt das Gewichtsverhältnis Allulose: Süßstoff im Bereich zwischen von etwa von etwa 10:90 bis 90:10, vorzugsweise von etwa 25:75 bis 75:25 und insbesondere etwa 40:60 bis 60:40.

Geschmacksverstärker, deren Wirkung zusätzlich verstärkt und/oder deren unangenehmen Geschmacksnoten (adstringierend, metallisch, teerig, fettig) überdeckt oder maskiert werden, umfassen beispielsweise: Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), weitere Hydroxyflavanone (z.B. Eriodictyol, Homoeriodictyol oder deren Natriumsalze), insbesondere gemäß US 2002/0188019, Hydroxybenzoesäureamide nach DE 10 2004 041 496 (z.B. 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)-amid, 2-Hydroxy-benzoesäure-N-4-(hydroxy-3-methoxybenzyl)amid, 4-Hydroxybenzoesäure-N-(4-hydroxy-3-methoxybenzyl)amid, 2,4-Dihydroxybenzoesäure-N-(4-hydroxy-3-methoxyben-zyl)amid-Mono-natriumsalz, 2,4-Dihydroxybenzoesäure-N-2-(4-hydroxy-3-methoxyphenyl)-ethylamid, 2,4-Dihydroxybenzoesäure-N-(4-hydroxy-3-ethoxybenzyl)amid, 2,4-Dihydroxy-benzoesäure-N-(3,4-dihy-droxybenzyl)amid und 2-Hydroxy-5-methoxy-N-[2-(4-hydroxy-3-methoxyphenyl)ethyl]amid (Aduncamid), 4-Hydroxybenzoesäurevanillylamid), bittermaskierende Hydroxydeoxybenzoine z.B. gemäß WO 2006/106023 (z.B. 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxy-phenyl)ethanon), Aminosäuren (z.B. gamma-Aminobuttersäure nach WO 2005/096841 zur Verminderung oder Maskierung eines unangenehmen Geschmackseindrucks wie Bitterkeit), Äpfelsäureglycoside nach WO 2006/003107, salzig schmeckende Mischungen gemäß PCT/EP 2006/067120 Diacetyltrimere gemäß WO 2006/058893, Gemische von Molkeproteinen mit Lecithinen und/oder bittermaskierende Substanzen wie Gingerdione gemäß WO 2007/003527.

Demzufolge ist ein weiterer Gegenstand der vorliegenden Erfindung die Verwendung von Allulose, insbesondere von kristalliner Allulose wie in den Absätzen [0017]-[0020] beschrieben, oder erhältlich durch das in den Absätzen [0025]-[0040] beschriebenen Verfahren, zur Überdeckung und/oder Maskierung der unangenehmen Geschmacksnoten (adstringierend, metallisch, teerig, fettig) von Geschmacksverstärkern, insbesondere der oben genannten Geschmacksverstärker.

In einer besonders bevorzugten Ausführungsform wird Allulose, insbesondere von kristalliner Allulose wie in den Absätzen [0017]-[0020] beschrieben, oder erhältlich durch das in den Absätzen [0025]-[0040] beschriebenen Verfahren, zur Maskierung der unangenehmen Geschmacksnoten (adstringierend, metallisch, teerig, fettig) von Geschmacksverstärkern, insbesondere der oben genannten Geschmacksverstärkern, verwendet. Bei einer solchen Verwendung liegt das Gewichtsverhältnis Allulose: Geschmacksverstärker im Bereich zwischen von etwa von etwa 10:90 bis 90: 10, vorzugsweise von etwa 25:75 bis 75:25 und insbesondere etwa 40:60 bis 60:40.

Bevorzugte Aromastoffe sind solche, die einen süßen Geruchseindruck verursachen, wobei der oder die weiteren Aromastoffe, die einen süßen Geruchseindruck verursachen, bevorzugt ausgewählt sind aus der Gruppe bestehend aus: Vanillin, Ethylvanillin, Ethylvanillinisobutyrat (= 3-Ethoxy-4-isobutyryloxybenzaldehyd), Furaneol® (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäure-allylester, n-Hexansäure-n-butylester, n-Octansäureethylester, Ethyl-3-methyl-3-phe-nylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al, 4-Hydroxyzimtsäure, 4-Methoxy-3-hydroxyzimtsäure, 3-Methoxy-4-hydroxyzimtsäure, 2-Hydroxyzimtsäure, 2,4-Dihydroxybenzoesäure, 3-Hydroxybenzoesäure, 3,4-Dihydroxybenzoesäure, Vanillinsäure, Homovanillinsäure, Vanillomandelsäure und Phenylacetaldehyd.

Demzufolge ist ein weiterer Gegenstand der vorliegenden Erfindung die Verwendung von Allulose, insbesondere von kristalliner Allulose wie in den Absätzen [0017]-[0020] beschrieben, oder erhältlich durch das in den Absätzen [0025]-[0040] beschriebenen Verfahren, zur Verstärkung der Süßkraft und/oder Überdeckung und/oder Maskierung der unangenehmen Geschmacksnoten (adstringierend, metallisch, teerig, fettig) von Aromastoffen, insbesondere der oben genannten Aromastoffen.

In einer besonders bevorzugten Ausführungsform wird Allulose, insbesondere von kristalliner Allulose wie in den Absätzen [0017]-[0020] beschrieben, oder erhältlich durch das in den Absätzen [0025]-[0040] beschriebenen Verfahren, zur Maskierung der unangenehmen Geschmacksnoten (adstringierend, metallisch, teerig, fettig) von Aromastoffen, insbesondere der oben genannten Aromastoffen, verwendet. Bei einer solchen Verwendung liegt das Gewichtsverhältnis Allulose: Geschmacksverstärker im Bereich zwischen von etwa von etwa 10:90 bis 90: 10, vorzugsweise von etwa 25:75 bis 75:25 und insbesondere etwa 40:60 bis 60:40.

Mund- und Zahnpflegemittel

Wie bereits erwähnt, kann die kristalline Allulose wie in den Absätzen [0017]-[0020] beschrieben, oder erhältlich durch das in den Absätzen [0025]-[0040] beschriebenen Verfahren zur Herstellung von Mund- und Zahnpflegemittel verwendet werden. Beispiele hierfür sind Zahnpasten, Zahngele, Zahnpulver, Mundwässer, (medizinische) Kaugummis und dergleichen. Die Produkte aus diesen Gruppen, weisen mindestens einen der folgenden Vorteile auf:

- Süßung ohne Kalorien und ohne E-Nummer;
- echter Zucker ohne Kalorien + ohne "Off-taste";
- 100% Saccharoseaustausch sowie beliebige Kombinationen möglich;
- geeignet für Diabetiker;
- verbessertes Geschmacksprofil;
- verbesserte Farbgebung;
- Verstärkung der Geschmacksintensität (z.B. intensiverer Minzgeschmack);
- Verstärkung der Kühlwirkung (z.B. länger anhaltender Mentholgeschmack); sowie
- geringere Schaumbildung.

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die Zahnpasten im Sinne der vorliegenden Erfindung können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxid-trihydrat eingesetzt werden.

Bevorzugt geeignete Putzkörper für die Zahnpasten Zahnpasten im Sinne der vorliegenden Erfindung sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummi arabicum, Xantham-Gum und Carboxyvinylpolymere (z. B. Carbopol®-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:

- Konservierungsmittel und antimikrobielle Stoffe wie zum Beispiel p- Hydroxybenzösäuremethyl-, - ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419, DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharoseharin-Natrium, Natrium-Cyclamat, Sucrose, Lactose, Maltose, Fructose oder Apartam®, (L-Aspartyl- L-phenylalanin-methylester), Stivia-extrakte oder deren sü-ßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- Wundheilende und entzündungshemmende Stoffe wie z. B. Allantoin, Harnstoff, Azulen, Kamillenwirkstoffe und Acetylsalicylsäurederivate.

Eine bevorzugte Ausführung der oralen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

In Mundwässern ist eine Kombination mit wässrig-alkoholischen Lösungen verschiedener Grädigkeit von ätherischen Ölen, Emulgatoren, adstringierenden und tonisierenden Drogenauszügen, zahnsteinhemmenden, antibakteriellen Zusätzen und Geschmackskorrigentien ohne weiteres möglich. Eine weitere bevorzugte Ausführung der Erfindung ist ein Mundwasser in Form einer wässrigen oder wässrig-alkoholischen Lösung enthaltend die Mischung aus Menthofuran und Mentholverbindungen in Mengen von 0,5 bis 2 Gew.-%. In Mundwässern, die vor der Anwendung verdünnt werden, können mit, entsprechend dem vorgesehenen Verdünnungsverhältnis, höheren Konzentrationen ausreichende Effekte erzielt werden.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschilderten Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind

- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen),

Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Roh stoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Bei den bevorzugten oralen Zubereitungen kann es sich auch um Kaugummis handeln. Diese Produkte enthalten typischerweise eine wasserunlösliche und eine wasserlösliche Komponente.

Die wasserunlösliche Basis, die auch als *"Gummibasis"* bezeichnet wird, umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten

(nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird.

Zusätzlich zu der wasserunlöslichen Gummibasis enthalten Kaugummizubereitungen regelmäßig einen wasserlöslichen Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als zusätzliche Süßstoffe neben der kristallinen Allulose kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharose-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharose und Saccharosesalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 Gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des Weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

PHARMAZEUTISCHE MITTEL

Wie bereits erwähnt, kann die kristalline Allulose wie in den Absätzen [0017]-[0020] beschrieben, oder erhältlich durch das in den Absätzen [0025]-[0040] beschriebenen Verfahren zur Herstellung von pharmazeutischen Mitteln verwendet werden. Die geeigneten pharmazeutischen Mittel umfassen feste, tablettierte oder dragierte Wirkstoffe und Wirkstoffmischungen sowie flüssige Produkte. Diese Produkte zeichnen sich vor allem dadurch aus, dass sie nicht kariogen und damit auch für Diabetiker geeignet sind. In flüssigen Formulierungen findet keine Auskristallisation statt. Die Tablettierung gelingt einfacher, außerdem lassen sich Tabletten mit Allulose, wie beispielsweise "Aspirin Plus C" infolge der höheren Löslichkeit auch ohne Wasser einnehmen.

Im Sinne der Erfindung werden in einer ersten Ausführungsform (Lutsch-)Tabletten und Dragees, also feste Verabreichungsformen angesprochen, die einen oder mehrere pharmazeutische Wirkstoffe zusammen mit einem Tablettierungsmittel und gegebenenfalls einem Sprengmittel enthalten, deren Natur jedoch unkritisch ist.

Die Erfindung betrifft die überraschende Erkenntnis, dass der unangenehme, adstringierende und/oder metallische Nachgeschmack, der vielen pharmazeutischen Wirkstoffen (insbesondere den so genannten "non-steroidal anti-inflammatory drugs", NSAID) gemeinsam ist und deren Einnahme erschwert, durch die kristalline Allulose überdeckt und/oder maskiert wird. Geeignete Wirkstoffe, für die diese Erkenntnis zutrifft sind - ohne dabei abschließend zu sein - die folgenden:

(1) ASPIRIN (Acetylsalicylsäure)

(2) MINOXIDIL (6-piperidin-1-ylpyrimidin-2,4-diamin-3-oxid)

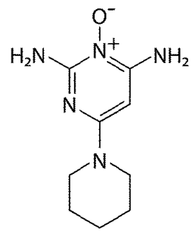

(3) ERYTHROMICIN

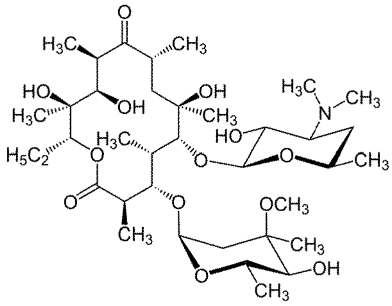

(4) DIMETINDENE (FENISTIL) (RS-dimethyl(2-(3-[pyridin-2-yl)ethyl]-1H-inden-2-yl)ethyl)amine)

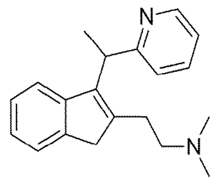

(5) BETAMETHOSON (8S,9R,10S.11S,13S,14S,16S,17R)-9-fluoro-11,17-(2-hydroxyacetyl)-10, 13,16-trimethyl-6,7,8,9,10,11,12,13,14,15,16,17-dodecahydro-3H-cyclopenta(alpha)-phenanthren-3-one

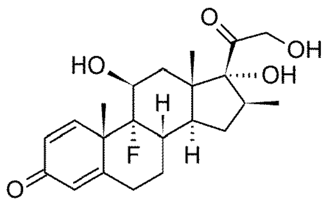

(6) IBUPROFEN (RS)-2-(4-(2-methylpropyl)phenyl)propanoic acid)

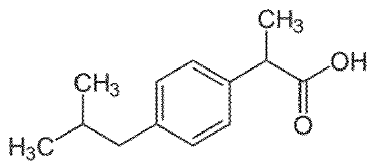

(7) KETOPROFEN (RS)2-(3-benzoylphenyl)-propionic acid)

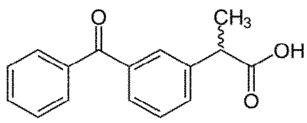

(8) DICLOFENAC

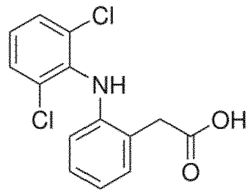

(9) Metronidazol (2-(2-methyl-5-nitro-1H-imidazol-1-yl)ethanol)

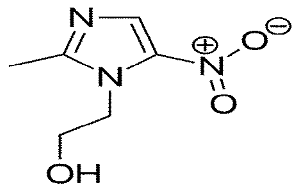

(10) ACYCLOVIR (2-Amino-1,9-dihydro-9-((2-hydroxyethoxy)methyl)-6H-Purin-6-one),

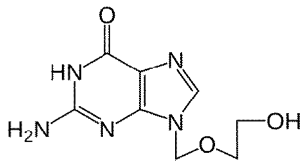

(11) IMIQUIMOD (3-(2-methylpropyl)-3,5,8-triazatricyclo[7.4.0.0.$^{2,6}$]trideca-1(9),2(6),4,7,10, 12-hexaen-7-amine

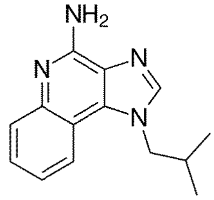

(12) TERBINAFIN [(2E)-6,6-dimethylhept-2-en-4-yn-1-yl](methyl)(naphthalen-1-ylmethyl)amine)

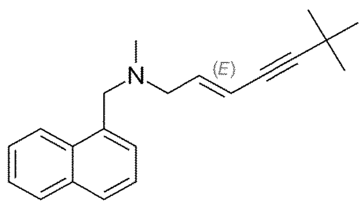

(13) CICLOPYRAXOLAMIN (6-cyclohexyl-1-hydroxy-4-methylpyridin-2(1H)-one)

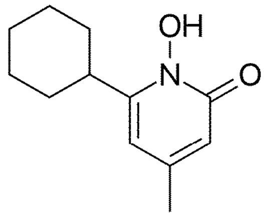

Demzufolge ist ein weiterer Gegenstand der vorliegenden Erfindung die Verwendung von Allulose, insbesondere von kristalliner Allulose wie in den Absätzen [0017]-[0020] beschrieben, oder erhältlich durch das in den Absätzen [0025]-[0040] beschriebenen Verfahren, zur Überdeckung und/oder Maskierung des unangenehmen, adstringierenden und/oder metallischen Nachgeschmacks von pharmazeutischen Wirkstoffen.

In einer besonders bevorzugten Ausführungsform wird Allulose, insbesondere von kristalliner Allulose wie in den Absätzen [0017]-[0020] beschrieben, oder erhältlich durch das in den Absätzen [0025]-[0040] beschriebenen Verfahren, zur Maskierung des unangenehmen, adstringierenden und/oder metallischen Nachgeschmacks von den so genannten "non-steroidal anti-inflammatory drugs"(NSAID) verwendet.

In einer weiteren besonders bevorzugten Ausführungsform wird Allulose, insbesondere von kristalliner Allulose wie in den Absätzen [0017]-[0020] beschrieben, oder erhältlich durch das in den Absätzen [0025]-[0040] beschriebenen Verfahren, zur Maskierung des unangenehmen, adstringierenden und/oder metallischen Nachgeschmacks von Wirkstoffen, ausgewählt aus der Gruppen bestehend aus ASPIRIN (Acetylsalicylsäure), MINOXIDIL (6-piperidin-1-ylpyrimidin-2,4-diamin-3-oxid), ERYTHROMICIN, DIMETINDENE (FENISTIL) (RS-dimethyl(2-(3-[pyridin-2-yl)ethyl]-1H-inden-2-yl)ethyl)amine), BETAMETHOSON (85,9R,10S.11S,13S,14S,16S,17R)-9-fluoro-11,17-(2-hydro-xyacetyl)-10, 13,16-trimethyl-6,7,8,9,10,11,12,13,14,15,16,17-dodecahydro-3H-cyclopenta(alpha)-phenanthren-3-one, IBUPROFEN (RS)-2-(4-(2-methylpropyl)phenyl)propanoic acid), KETOPROFEN (RS)2-(3-benzoylphenyl)-propionic acid), DICLOFENAC, Metronidazol (2-(2-methyl-5-nitro-1H-imidazol-1-yl)ethanol, ACYCLOVIR (2-Amino-1,9-dihydro-9-((2-hydroxyethoxy)methyl)-6H-Purin-6-one), IMIQUIMOD (3-(2-methylpropyl)-3,5,8-triazatricyclo[7.4.0.0.2,6]trideca-1(9),2(6),4,7,10, 12-hexaen-7-amine, TERBINAFIN [(2E)-6,6-dimethylhept-2-en-4-yn-1-yl](methyl)(naphthalen-1-ylme-thyl)amine), CICLOPYRAXOLAMIN (6-cyclohexyl-1-hydroxy-4-methylpyridin-2(1H)-one) oder deren Mischungen, verwendet.

Bei einer der oben genannten Verwendungen liegt das Gewichtsverhältnis Allulose: Wirkstoff im Bereich zwischen von etwa von etwa 10:90 bis 90:10, vorzugsweise von etwa 25:75 bis 75:25 und insbesondere etwa 40:60 bis 60:40.

Neben den festen Zubereitungen können auch flüssige Produkte unter Verwendung die kristallinen Allulose wie in den Absätzen [0017]-[0020] beschrieben, oder erhältlich durch das in den Absätzen [0025]-[0040] beschriebenen Verfahren hergestellt werden, wie beispielsweise Hustensaft oder Sprayformulierungen.

Bevorzugte Ausführungsformen der Erfindung werden durch nachfolgende Sätze definiert:

Satz 1: Allulose in kristalliner Form, dadurch gekennzeichnet, dass sie eine Partikelgrößenverteilung bestimmt nach der $X_{c(min)}$-Methode aufweist, für die eine, zwei oder alle drei der folgenden Auswahlregeln gelten:

| Dispergierdruck [kPa] | Minimum d10 [μm] | Minimum d50 [μm] | Minimum d90 [μm] |
|---|---|---|---|
| 5 | > 70 | > 140 | > 230 |
| 20 | > 50 | > 120 | > 220 |
| 460 | > 10 | > 40 | > 130 |

und/oder

eine Partikelgrößenverteilung bestimmt nach der $X_{Fe(max)}$-Methode aufweist, für die eine, zwei oder alle drei der folgenden Auswahlregeln gelten:

| Dispergierdruck [kPa] | Minimum d10 [μm] | Minimum d50 [μm] | Minimum d90 [μm] |
|---|---|---|---|
| 5 | > 100 | > 220 | > 340 |
| 20 | > 70 | > 200 | > 330 |
| 460 | > 8 | > 65 | > 170 |

und/oder
eine Partikelgrößenverteilung bestimmt nach der $X_{area}$-Methode aufweist, für die eine, zwei oder alle drei der folgenden Auswahlregeln gelten:

| Dispergierdruck [kPa] | Minimum d10 [μm] | Minimum d50 [μm] | Minimum d90 [μm] |
|---|---|---|---|
| 5 | > 85 | > 185 | > 265 |
| 20 | > 65 | > 180 | > 260 |
| 460 | > 10 | > 50 | > 120 |

Satz 2: Allulose nach Satz 1, dadurch gekennzeichnet, dass diese einen b/l d50-Wert aufweist, für den gilt: und/oder

| Parameter | Dispergierdruck [kPa] | $X_{c(min)}$ |
|---|---|---|
| b/l d50 | 5 | 0,52-1,0 |
| und/oder | | |
| b/l d50 | 20 | 0,53-1,0 |
| und/oder | | |
| b/l d50 | 460 | 0,60 - 1,0 |

und/oder

| Parameter | Dispergierdruck [kPa] | $X_{Fe(max)}$ |
|---|---|---|
| b/l d50 | 5 | 0,52-1,0 |
| und/oder | | |
| b/l d50 | 20 | 0,55-1,0 |
| und/oder | | |
| b/l d50 | 460 | 0,65-1,0 |

| Parameter | Dispergierdruck [kPa] | $X_{area}$ |
|---|---|---|
| b/l d50 | 5 | 0,52-1,0 |
| und/oder | | |
| b/l d50 | 20 | 0,55-1,0 |
| und/oder | | |
| b/l d50 | 460 | 0,65-1,0 |

Satz 3: Verfahren zur Herstellung von kristalliner Allulose gemäß mindestens einem der vorstehenden Sätze 1 bis 2, umfassend oder bestehend aus folgenden Schritten:

(a) Bereitstellen einer wässrigen Allulose-Zubereitung, vorzugsweise einem Verdampferkonzentrat ("Allulose-Sirup"), umfassend oder bestehend aus folgenden Komponenten, bezogen auf die in der Zubereitung enthaltene Trockenmasse:

(i) etwa 90 bis etwa 99 Gew.-% Allulose,
(ii) etwa 0,5 bis etwa 10 Gew.-% Fructose,
(iii) 0 bis etwa 5 Gew.-% weitere Kohlenhydrate, verschieden von Allulose und Fructose,

mit der Maßgabe, dass sich die Mengenangaben zu 100 Gew.-% ergänzen;

(b) optionale Behandlung der Allulose-Zubereitung des Schrittes (a) mit einem Entfärbungsmittel;
(c) Aufkonzentrieren der Zubereitung aus Schritt (a) oder Schritt (b) bis die Sättigungskonzentration überschritten wird;
(c) Versetzen der übersättigten Zubereitung aus Schritt (c) mit Allulose-Impfkristallen; und
(d) Abkühlen der Zubereitung aus Schritt (c) mit einer Geschwindigkeit von 0,1 bis etwa 1 K/h bis ein Kristallgehalt von etwa 30 bis etwa 50 Gew.-% erreicht wird, sowie
(e) Abtrennen und optional Trocknen der Kristalle.

Satz 4: Verfahren nach Satz 3, dadurch gekennzeichnet, dass die Zubereitung des Schrittes (a) einen Gehalt an Allulose ≥ 90 Gew.-%, vorzugsweise ≥ 91 Gew.-%, vorzugsweise ≥ 92 Gew.-%, vorzugsweise ≥ 93 Gew.-%, vorzugsweise ≥ 94 Gew.-%, vorzugsweise ≥ 95 Gew.-%, vorzugsweise ≥ 96 Gew.-%, vorzugsweise ≥ 97 Gew.-%, vorzugsweise ≥ 98 Gew.-%, jeweils bezogen auf die in der Zubereitung enthaltene Trockenmasse, aufweist.
Satz 5: Verfahren nach Satz 3, dadurch gekennzeichnet, dass die Zubereitung des Schrittes (a) einen Gehalt an Allulose ≤ 99 Gew.-%, vorzugsweise ≤ 98 Gew.-%, vorzugsweise ≤ 97 Gew.-%, vorzugsweise ≤ 96 Gew.-%, vorzugsweise ≤ 95 Gew.-%, vorzugsweise ≤ 94 Gew.-%, vorzugsweise ≤ 93 Gew.-%, vorzugsweise ≤ 92 Gew.-%, vorzugsweise ≤ 91 Gew.-%, jeweils bezogen auf die in der Zubereitung enthaltene Trockenmasse, aufweist.
Satz 6: Verfahren nach Satz 3, dadurch gekennzeichnet, dass die Zubereitung des Schrittes (a) einen Gehalt an Fructose von etwa 1 bis etwa 3 Gew.-% Fructose, bezogen auf die in der Zubereitung enthaltene Trockenmasse, aufweist.
Satz 7: Verfahren nach Satz 6, dadurch gekennzeichnet, dass die Zubereitung des Schrittes (a) einen Gehalt an Fructose von etwa 1,5 bis etwa 2,5 Gew.-% Fructose, bezogen auf die in der Zubereitung enthaltene Trockenmasse, aufweist.
Satz 8: Verfahren nach Satz 3, dadurch gekennzeichnet, dass die Zubereitung des Schrittes (a) einen Gehalt an Fructose von etwa 0,5 bis etwa 1 Gew.-% Fructose, oder von etwa 1 bis etwa 1,5 Gew.-%, oder von etwa 1,5 bis etwa 2,0 Gew.-%, oder von etwa 2,0 bis etwa 2,5 Gew.-%, oder von etwa 2,5 bis etwa 3,0 Gew.-%, oder von etwa 3,0 bis etwa 3,5 Gew.-%, oder von etwa 3,5 bis etwa 4,0 Gew.-%, oder von etwa 4,0 bis etwa 4,5 Gew.-%, oder von etwa 4,5 bis etwa 5,0 Gew.-%, oder von etwa 5,0 bis etwa 5,5 Gew.-%, oder von etwa 5,5 bis etwa 6,0 Gew.-%, oder von etwa 6,0 bis etwa 6,5 Gew.-%, oder von etwa 6,5 bis etwa 7,0 Gew.-%, oder von etwa 7,0 bis etwa 7,5 Gew.-%, oder von etwa 7,5 bis etwa 8,0 Gew.-%, oder von etwa 8,0 bis etwa 8,5 Gew.-%, oder von etwa 8,5 bis etwa 9,0 Gew.-%, oder von etwa 9,5 bis etwa 10 Gew.-%, jeweils bezogen auf die in der Zubereitung enthaltene Trockenmasse, aufweist.
Satz 9: Verfahren nach mindestens einem der Sätze 3 bis 8, dadurch gekennzeichnet, dass man im Schritt (a) Zubereitungen einsetzt, die einen Trockensubstanzgehalt von etwa 55 bis etwa 98 Gew.-% aufweisen.
Satz 10: Verfahren nach Satz 9, dadurch gekennzeichnet, dass man im Schritt (a) Zubereitungen einsetzt, die einen Trockensubstanzgehalt von etwa 75 bis etwa 95 Gew.-% aufweisen.
Satz 11: Verfahren nach Satz 10, dadurch gekennzeichnet, dass man im Schritt (a) Zubereitungen einsetzt, die einen Feststoffgehalt von etwa 75 bis etwa 80 Gew.-%, oder von etwa 80 bis etwa 85 Gew.-%, von etwa 85 bis etwa 90 Gew.-%, von etwa 90 bis etwa 95 Gew.-%, aufweisen.
Satz 12: Verfahren nach mindestens einem der Sätze 3 bis 11, dadurch gekennzeichnet, dass die Aufkonzentrierung des Schrittes (c) bei einer Temperatur von etwa 50 bis etwa 75 °C durchgeführt wird.
Satz 13: Verfahren nach mindestens einem der Sätze 3 bis 12, dadurch gekennzeichnet, dass man der übersättigten Zubereitung Impfkristalle in Mengen von etwa 1 bis etwa 3 Gew.-% - bezogen auf die Zubereitung - zusetzt.
Satz 14: Verwendung von kristalliner Allulose nach mindestens einem der Sätze 1 bis 2 oder erhältlich durch das Verfahren nach mindestens einem der Sätze 3 bis 13 zur Herstellung von oralen Zubereitungen.
Satz 15: Verwendung nach Satz 14, dadurch gekennzeichnet, dass die oralen Zubereitungen ausgewählt sind aus der Gruppe, die gebildet wird von Nahrungsmitteln, Mund- und Zahnpflegemitteln sowie pharmazeutischen Zubereitungen.
Satz 16: Verwendung nach Satz 15, dadurch gekennzeichnet, dass die Nahrungsmittel ausgewählt sind aus der Gruppe, die gebildet wird von Getränken, Backwaren, Getreideprodukten, Knabberartikeln, Süßwaren, Milchprodukten, Fruchtzubereitungen, Würzmitteln, Gemüsezubereitungen, Fleischwaren, Gewürzen und Gewürzmischungen.
Satz 17: Verwendung nach Satz 16, dadurch gekennzeichnet, dass die Getränke ausgewählt sind aus der Gruppe, die gebildet wird von Kaffee, Tee, Eistee, Wein, weinhaltigen Getränke, Bier, bierhaltigen Getränken, Likören, Schnäpsen, Weinbränden, (karbonisierten) fruchthaltigen Limonaden, (karbonisierten) isotonischen Getränken, mit Geschmacksstoffen angereichertem Mineralwasser, (karbonisierten) Erfrischungsgetränken, Nektaren, Schorlen, Obst- und Gemüsesäften, Frucht- oder Gemüsesaftzubereitungen, Instant-Kakao-Getränken, Instant-Tee-Getränken, Instant-Kaffeegetränken und Instant-Fruchtgetränken.
Satz 18: Verwendung nach Satz 16, dadurch gekennzeichnet, dass die Backwaren, Getreideprodukte und Knabberartikel ausgewählt sind aus der Gruppe, die gebildet wird von Brot, Trockenkeksen, Kuchen, Muffins, Donuts, sonstigem Gebäck, gebackenen oder frittierten Kartoffelchips oder Kartoffelteigprodukten, Extrudaten auf Mais- oder Erdnussbasis, Frühstückscerealien, Müsliriegeln und vorgegarten Fertigreis-Produkten.
Satz 19: Verwendung nach Satz 16, dadurch gekennzeichnet, dass die Süßwaren und Fruchtzubereitungen ausgewählt sind aus der Gruppe, die gebildet wird von Schokoladen, Schokoladenriegelprodukten, sonstige Riegelprodukten,

Fruchtgummis, Hart- und Weichkaramellen, Kaugummis, Tabletten, Mints, Überzügen (Fondants), Konfitüren, Fruchteis, Fruchtsoßen und Fruchtfüllungen.

Satz 20: Verwendung nach Satz 16, dadurch gekennzeichnet, dass die Milchprodukte ausgewählt sind aus der Gruppe, die gebildet wird von Milchgetränken, Buttermilchgetränken, Milcheis, Joghurt, Kefir, Frischkäse, Weichkäse, Hartkäse, Trockenmilchpulvern, Molke, Molkegetränken, Butter, Buttermilch, teilweise oder ganz hydrolysierte Milchprotein-haltigen Produkten, Produkte aus Sojaprotein oder anderen Sojabohnen-Fraktionen, Fruchtgetränken mit Sojaprotein, Sojalecithin-haltige Zubereitungen, fermentierte Produkte wie Tofu oder Tempe oder daraus gefertigte Produkte) und Produkten aus anderen pflanzlichen Proteinquellen.

Satz 21: Verwendung nach Satz 16, dadurch gekennzeichnet, dass die Würzmittel und Gemüsezubereitungen ausgewählt sind aus der Gruppe, die gebildet wird von Ketchup, Soßen, Trockengemüsen, Tiefkühlgemüsen, vorgegarten Gemüsen, eingekochten Gemüsen, Mayonnaise, Remoulade, Dressings, Fertiggerichten, Trockensuppen, Instant-Suppen, vorgegarte Suppen, Gewürzen, Würzmischungen und Aufstreuwürzen (englisch: Seasonings).

Satz 22: Verwendung nach Satz 16, dadurch gekennzeichnet, dass die Fleischprodukte ausgewählt sind aus der Gruppe, die gebildet wird von Schinken, Frischwurst- oder Rohwurstzubereitungen, und gewürzten oder marinierten Frisch- oder Pökelfleischprodukten.

Satz 23: Verwendung nach Satz 16, dadurch gekennzeichnet, dass die Gewürze und Gewürzmischungen ausgewählt sind aus der Gruppe, die gebildet wird von Süßkraftverstärkern, Geschmacksverstärkern und Maskierungsmitteln.

Satz 24: Verwendung nach Satz 15, dadurch gekennzeichnet, dass die Mund- und Zahnpflegemittel ausgewählt sind aus der Gruppe, die gebildet wird von Zahnpasten, Mundwässern und Kaugummis.

Satz 25: Verwendung nach Satz 15, dadurch gekennzeichnet, dass die pharmazeutischen Mittel ausgewählt sind aus der Gruppe, die gebildet wird von festen, tablettierten oder dragierten Wirkstoffen und Wirkstoffmischungen sowie flüssigen Produkten.

Satz 26: Verwendung von Allulose zur Überdeckung und/oder Maskierung des unangenehmen, adstringierenden und/oder metallischen Nachgeschmacks von pharmazeutischen Wirkstoffen.

Satz 27: Verwendung nach Satz 26, dadurch gekennzeichnet, dass es sich bei den pharmazeutischen Wirkstoffen um "non-steroidal anti-inflammatory drugs"(NSAID) handelt.

Satz 28: Verwendung nach Satz 26, dadurch gekennzeichnet, dass der pharmazeutische Wirkstoff aus der Gruppen bestehend aus ASPIRIN (Acetylsalicylsäure), MINOXIDIL (6-piperidin-1-ylpyrimidin-2,4-diamin-3-oxid), ERYTHROMICIN, DIMETINDENE (FENISTIL) (RS-dimethyl(2-(3-[pyridin-2-yl)ethyl]-1H-inden-2-yl)ethyl)amine), BETAMETHOSON (8S,9R,10S.11S,13S,14S,16S,17R)-9-flu-oro-11,17-(2-hydroxyacetyl)-10, 13,16-trimethyl-6,7,8,9,10,11,12,13,14,15,16,17-dodecahydro-3H-cyclopenta(alpha)-phenanthren-3-one, IBUPROFEN (RS)-2-(4-(2-methylpropyl)phenyl)propanoic acid), KETOPROFEN (RS)2-(3-benzoylphenyl)-propionic acid), DICLOFENAC, Metronidazol (2-(2-methyl-5-nitro-1H-imidazol-1-yl)ethanol, ACYCLOVIR (2-Amino-1,9-dihydro-9-((2-hydro-xyethoxy)methyl)-6H-Purin-6-one), IMIQUIMOD (3-(2-methylpropyl)-3,5,8-tria-zatricyclo[7.4.0.0.2,6]trideca-1(9),2(6),4,7,10, 12- hexaen-7-amine, TERBINAFIN [(2E)-6,6-dimethyl-hept-2-en-4-yn-1-yl](methyl)(naphthalen-1-ylmethyl)amine), CICLOPYRAXOLAMIN (6-cyclohexyl-1-hydroxy-4-methylpyridin-2(1H)-one) oder deren Mischungen ausgewählt ist.

Satz 29: Verwendung von Allulose, zur Überdeckung und/oder Maskierung der unangenehmen Geschmacksnoten (adstringierend, metallisch, teerig, fettig) von Geschmacksverstärkern.

Satz 30: Verwendung von Allulose zur Verstärkung der Süßkraft und/oder Überdeckung und/oder Maskierung der unangenehmen Geschmacksnoten (adstringierend, metallisch, teerig, fettig) von Aromastoffen.

BEISPIELE

Die vorliegende Erfindung wird unter Bezugnahme auf die nachstehenden Beispiele leichter zu verstehen sein.

Jedoch dienen diese Beispiele lediglich zur Veranschaulichung der Erfindung und können nicht als einschränkend in Bezug auf den Schutzbereich der Erfindung ausgelegt werden. Alle Mengenangaben - wenn nicht anders angegeben - verstehen sich als Gew.-%.

BEISPIEL 1 (ERFINDUNGSGEMÄSS): *Kristallisation von Allulose-Lösungen*

1.000 Liter einer wässrigen Allulose-Zubereitung mit einem Trockensubstanzgehalt von 70 Gew.-% und einem Gehalt an Allulose von 92 Gew.-% und einem Gehalt an Fructose von 1,8 Gew.-%, bezogen auf die in der Zubereitung enthaltene Trockenmasse, wurde mit 1 Gew.-% Aktivkohle versetzt und über 3 Stunden gerührt.
Anschließend wurde die entfärbte Lösung filtriert und aufkonzentriert, bis eine übersättigte Lösung erhalten wurde.
Diese wurde in einen Kristallisator überführt und mit 1,5 Gew.-% Allulosekristallen geimpft. Danach wurde das Filtrat mit einer Geschwindigkeit von 1 K/h abgekühlt, bis sich die ersten Kristalle abschieden. Die Kristallisationsgeschwindigkeit wurde auf 0,5 K/h reduziert bis eine Kristallmenge von etwa 45 Gew.-% bezogen auf die Trockenmasse der übersättigten

Lösung erreicht war.
Anschließend wurden die Kristalle abgetrennt und getrocknet; die Mutterlauge wurde in den Prozess zurückgeführt. Es wurde eine kristalline Allulose erhalten, die die folgende Partikelgrößenverteilung aufwies:

| Parameter | Dispergierdruck [kPa] | $X_{c(min)}$ | $X_{Fe(max)}$ | $X_{area}$ |
|---|---|---|---|---|
| Minimum d10 [µm] | 5 | 72 | 105 | 90 |
| Minimum d50 [µm] | 5 | 150 | 230 | 190 |
| Minimum d90[µm] | 5 | 235 | 350 | 270 |
| Minimum d10 [µm] | 20 | 55 | 75 | 75 |
| Minimum d50 [µm] | 20 | 125 | 220 | 195 |
| Minimum d90[µm] | 20 | 225 | 335 | 280 |
| Minimum d10 [µm] | 460 | 12 | 10 | 12 |
| Minimum d50 [µm] | 460 | 45 | 70 | 55 |
| Minimum d90[µm] | 460 | 135 | 175 | 135 |

VERGLEICHSBEISPIEL V1: *Kristallisation von Allulose-Lösungen*

1.000 Liter einer wässrigen Allulose-Zubereitung mit einem Trockensubstanzgehalt von 70 Gew.-% und einem Gehalt an Allulose von 92 Gew.-% und einem Gehalt an Fructose von 1,8 Gew.-%, bezogen auf die in der Zubereitung enthaltene Trockenmasse, wurde mit 1 Gew.-% Aktivkohle versetzt und über 3 Stunden gerührt.
Anschließend wurde die entfärbte Lösung filtriert und aufkonzentriert, bis eine übersättigte Lösung erhalten wurde. Diese wurde in einen Kristallisator überführt. Danach wurde das Filtrat mit einer Geschwindigkeit von 5 K/h abgekühlt, bis sich die ersten Kristalle abschieden. Die Kristallisationsgeschwindigkeit wurde auf 1 K/h reduziert bis eine Kristallmenge von etwa 45 Gew.-% bezogen auf die Trockenmasse der übersättigten Lösung erreicht war.
Anschließend wurden die Kristalle abgetrennt und getrocknet; die Mutterlauge wurde in den Prozess zurückgeführt. Es wurde eine kristalline Allulose erhalten, die die folgende Partikelgrößenverteilung aufwies:

| Parameter | Dispergierdruck [kPa] | $X_{c(min)}$ | $X_{Fe(max)}$ | $X_{area}$ |
|---|---|---|---|---|
| Minimum d10 [µm] | 5 | 60 | 90 | 75 |
| Minimum d50 [µm] | 5 | 130 | 210 | 175 |
| Minimum d90 [µm] | 5 | 215 | 320 | 255 |
| Minimum d10 [µm] | 20 | 40 | 60 | 50 |
| Minimum d50 [µm] | 20 | 110 | 185 | 170 |
| Minimum d90 [µm] | 20 | 210 | 315 | 250 |
| Minimum d10 [µm] | 460 | 2 | 1 | 2 |
| Minimum d50 [µm] | 460 | 30 | 55 | 40 |
| Minimum d90 [µm] | 460 | 110 | 150 | 110 |

BEISPIEL 2, VERGLEICHSBEISPIELE V2 UND V3: *Bestimmung des Wasserwertes*

Es wurden gleichartige Backwaren (Brownies) unter Verwendung von Saccharose (V2), erfindungsgemäßer kristalliner Allulose nach Beispiel 1 sowie kristalliner nach Vergleichsbeispiel V1 hergestellt. Jeweils Proben von 20 g der drei Produkte wurden Behälter gegeben, hermetisch verschlossen und dann über 3 h bei 20 °C gelagert. Anschließend wurde mit einem Hygrometer die Luftfeuchtigkeit bestimmt, die sich im Gasraum über den Proben eingestellt hatte. Die Zusammensetzung der Produkte sowie die Luftfeuchtigkeitswerte sind in der nachfolgenden Tabelle A wiedergegeben.

Tabelle A Zusammensetzung und Luftfeuchtigkeit

| Zusammensetzung | 2 | V2 | V3 |
|---|---|---|---|
| Vollei | 240,0 g | 240,0 g | 240,0 g |
| Salz | 2,0 g | 2,0 g | 2,0 g |
| Allulose gemäß Beispiel 1 | 260,0 g | - | - |
| Allulose gemäß Beispiel V1 | - | 260,0 g | - |
| Saccharose | - | - | 260,0 g |
| Butter | 230,0 g | 230,0 g | 230,0 g |
| Mehl Typ 405 | 70,0 g | 70,0 g | 70,0 g |
| Kakaopulver | 110,0 g | 110,0 g | 110,0 g |
| Aroma (Vanille) | 5,0 g | 5,0 g | 5,0 g |
| *Luftfeuchtigkeit [%]* | | | |
| - nach 6 h | 72 | 76 | 80 |
| - nach 48 h | 71 | 75 | 75 |

Die Beispiele und Vergleichsbeispiele zeigen, dass mit der neuen Allulosequalität der Wasserwert in Lebensmitteln gesenkt und damit deren Haltbarkeit verbessert werden kann.

BEISPIEL 3: *Geschmackliche Eigenschaften von Softdrinks*

Verschiedene Stoffgemische auf Basis von Steviolglykosiden (Rebaudiosid A) mit und ohne Allulose-Kristallen wurden zur Herstellung von einfachen Softdrinks verwendet und die Produkte 48 h bei 20°C gelagert. Anschließend wurden die geschmacklichen Eigenschaften (Deskriptoren: Anfangssüße, Süßintensität, Zuckergeschmack/Mundfülle) von einem Panel bestehend aus 8 geschulten Testern auf einer Linien-Skala von 0 (nicht vorhanden) bis 10 (stark ausgeprägt) bewertet. Die Zusammensetzungen und Ergebnisse sind in der nachfolgenden Tabelle 1 zusammengefasst. Die Ausführungen 3 und 4 sind erfindungsgemäß, die Beispiele V4 bis V6 dienen zum Vergleich. Das Beispiel C entspricht dem Standard, d.h. der geschmacklichen Beurteilung des Produktes ohne Zusatz von Stevia.

Tabelle 1

| Geschmackliche Eigenschaften von Softdrinkformulierungen | | | | | | |
|---|---|---|---|---|---|---|
| Zusammensetzung | C | 3 | 4 | V4 | V5 | V6 |
| Allulose | - | 7,0 | 10,0 | - | - | - |
| Saccharose | 10,0 | - | - | 7,0 | 8,0 | 10,0 |
| Zitronensäure | 0.2 | 0.2 | 0.2 | 0.2 | 0.2 | 0.2 |
| Rebaudiosid A | - | 0,0045 | 0,0045 | 0,0045 | 0,0045 | 0,0045 |
| Zitronenaroma | 0,1 | 0,1 | 0,1 | 0,1 | 0,1 | 0,1 |
| Wasser | Ad 100 | | | | | |
| *Geschmackliche Beurteilung* | | | | | | |
| Anfangssüße | 3,0 | 5,5 | 5,6 | 3,2 | 3,3 | 4,2 |
| Süßintensität | 3,1 | 6,3 | 6,4 | 3,5 | 3,8 | 4,5 |
| Zuckergeschmack und Mundfülle | 3,0 | 5,9 | 5,5 | 3,0 | 3,3 | 3,5 |
| Bitterkeit beim Nachgeschmack | 0,3 | 0,2 | 0,2 | 0,5 | 0,4 | 0,2 |

Die beiden erfindungsgemäßen Zubereitungen zeigen sowohl gegenüber dem Standard als auch den Stevia-haltigen Zubereitungen mit Sucrose Vorteile bezüglich Anfangssüße, Süßintensität, Zuckergeschmack, Mundfülle und Bitterkeit beim Nachgeschmack.

Im Folgenden wird die Erfindung an Hand weiterer Formulierungsbeispiele illustriert. Bei der Allulose handelt es sich um ein kristallines Produkt, welches die bevorzugten Auswahlregeln zur Partikelgrößenverteilung - wie oben beschrieben - erfüllt und von der Savanna Ingredients GmbH erhältlich ist.

FORMULIERUNGSBEISPIEL 1 Erfrischungsgetränke

| Zusammensetzung | 1.1 | 1.2 | 1.3 | 1.4 | 1.5 | 1.6 | 1.7 |
|---|---|---|---|---|---|---|---|
| Allulose | 10,0 | 10,0 | 7,0 | - | 10,0 | 8,0 | 7,0 |
| Rebaudiosid A | 0,0045 | 0,005 | 0,003 | 0,01 | 0,0045 | 0,0025 | 0,001 |
| Zitronensäure | 0,15 | 0,15 | 0,06 | 0,15 | 0,15 | 0,15 | 0,15 |
| Phosphorsäure | - | - | 0,07 | - | - | - | - |
| Zuckercouleur | - | - | 0,14 | - | - | - | - |
| Koffein | - | - | 0,01 | - | - | - | - |
| Zitrusaroma | 0,1 | 0,05 | - | 0,1 | - | 0,1 | 0,1 |
| Limonenaroma | - | 0,05 | - | - | 0,1 | - | - |
| Getränkeemulsion Typ "Cola" | - | - | 0,05 | - | - | - | - |
| Wasser | ad 100 | | | | | | |

FORMULIERUNGSBEISPIEL 2 Eistee

| Zusammensetzung | 2.1 | 2.2 |
|---|---|---|
| Schwarztee-Extrakt | 1,4 | 1,4 |
| Wasser | ad 100 | |
| Aromazubereitung (Typ Pfirsich) | 0,65 | 0,65 |
| Zucker | 3,5 | 3,0 |
| Zitronensäure (kristallin) | 1,2 | 1,2 |
| Ascorbinsäure | 0,2 | 0,2 |
| Allulose | 3,5 | 4,0 |

FORMULIERUNGSBEISPIEL 3 Tee Instantgetränk

| Zusammensetzung | 3.1 | 3.2 | 3.3 |
|---|---|---|---|
| schwarzer Tee, Ceylon, Blattware | 94,00 | - | - |
| grüner Tee, China, Blattware | - | 92,0 | - |
| Mate Tee, Peru, Blattware | - | - | 95,0 |
| Allulose | 6,0 | 8,0 | 5,0 |

FORMULIERUNGSBEISPIEL 4 Cappuccino Instantgetränk

| Zusammensetzung | 4.1 | 4.2 |
|---|---|---|
| Kaffee-Extrakt, sprühgetrocknet | 14,0 | 16,0 |
| Zucker | 25,0 | 20,0 |
| Allulose | 6,0 | 11,0 |
| Fettpulver | 18,2 | 18,2 |
| Kaffeeweißer, schäumend | ad100 | |
| Hydrokolloide/ Emulgatoren | 1,8 | 1,8 |
| Lactose | 4,7 | 4,7 |

FORMULIERUNGSBEISPIEL 5 Bittere Schokolade

| Zusammensetzung | 5.1 | 5.2 |
|---|---|---|
| Kakaomasse | ad 100 | |
| Kakaobutter | 11,70 | 11,70 |
| Zucker | 21,00 | 15,00 |
| Allulose | 9,00 | 15,00 |
| Vollmilch | 3,00 | 3,00 |
| Lezithin | 0,2 | 0,2 |
| Vanillin | 0,035 | 0,035 |

FORMULIERUNGSBEISPIEL 6 Fruchtgummis

| Zusammensetzung | 6.1 | 6.2 |
|---|---|---|
| Allulose | 34,50 | 8,20 |
| Rebaudiosid A | 0,003 | 0,003 |
| Iso Sirup C* Tru Sweet 01750 (Cerestar GmbH) | 1,50 | 2,10 |
| Gelatine 240 Bloom | 8,20 | 9,40 |
| Polydextrose (Litesse® Ultra, Danisco Cultor GmbH) | - | 24,40 |
| Farbstoff | 0,01 | 0,01 |
| Zitrusaroma | 0,20 | - |
| Kirscharoma | - | 0,10 |
| Wasser | ad 100 | |

FORMULIERUNGSBEISPIEL 7 Zuckerfreie Lutschbonbons

| Zusammensetzung | 7.1 | 7.2 | 7.3 |
|---|---|---|---|
| Wasser | 2,24 | 2,24 | 2,24 |
| Isomalt | ad 100 | | |
| Allulose | 2,40 | 2,60 | 3,0 |
| Sucralose | 0,03 | 0,03 | 0,03 |
| Acesulfam K | 0,050 | 0,050 | 0,050 |
| Zitronensäure | 0,050 | 0,050 | 0,050 |

FORMULIERUNGSBEISPIEL 8 Zahnpasten

| Zusammensetzung | 8.1 | 8.2 | 8.3 |
|---|---|---|---|
| Gum base | ad 100 | | |
| Sorbit, gepulvert | 40,00 | 40,00 | 40,00 |
| Isomalt, gepulvert | 9,50 | 9,50 | 9,50 |
| Xylit | 2,00 | 1,00 | 1,00 |
| Mannit D | 2,00 | 3,00 | 2,00 |
| Allulose | 1,00 | 1,00 | 1,00 |
| Acesulfam K | 0,10 | 0,10 | 0,10 |
| Emulgum / Plastifizierungsmittel | 0,30 | 0,30 | 0,30 |
| Sorbit (70% Wasser) | 13,00 | 13,00 | 13,00 |
| Glycerin | 1,00 | 1,00 | 1,00 |

FORMULIERUNGSBEISPIEL 9 Zahnpasten

| Zusammensetzung | 9.1 | 9.2 | 9.3 |
|---|---|---|---|
| Entionisiertes Wasser | 36,39 | 36,39 | 36,39 |
| Glycerin | 20,00 | 20,00 | 20,00 |
| Solbrol M (Natriumsalz) | 0,15 | 0,15 | 0,15 |
| Natriummonofluorphosphat | 0,76 | 0,76 | 0,76 |
| Allulose | 0,20 | 0,25 | 0,30 |
| Dicalciumphosphat-Dihydrat | 36,00 | 36,00 | 36,00 |
| Aerosil 200 | 3,00 | 3,00 | 3,00 |
| Natriumcarboxymethylcellulose | 1,20 | 1,20 | 1,20 |
| Natriumlaurylsulfat | 1,30 | 1,30 | 1,30 |

FORMULIERUNGSBEISPIEL 10 Mundwasserkonzentrat

| Zusammensetzung | 10.1 | 10.2 | 10.3 |
|---|---|---|---|
| Ethanol 96% | 42,00 | 42,00 | 42,00 |
| Cremophor RH 455 | 5,00 | 5,00 | 5,00 |
| Entionisiertes Wasser | 50,67 | 50,67 | 50,67 |
| Allantoin | 0,20 | 0,20 | 0,20 |
| Allulose | 0,10 | 0,20 | 0,30 |
| Colour L-Blue 5000 (1 % in Wasser) | 0,03 | 0,03 | 0,03 |
| Aroma | 2,00 | 2,00 | 2,00 |

FORMULIERUNGSBEISPIEL 11 Kaugummi

| Zusammensetzung | 11.1 | 11.2 |
|---|---|---|
| Kaugummibase, Company "Jagum T" | 30,00 | 30,00 |
| Sorbit, pulverisiert | 39,00 | 39,00 |
| Isomalt® (Palatinit GmbH) | 9,50 | 9,50 |
| Allulose | 2,00 | 2,00 |
| Mannit | 3,00 | 3,00 |
| Aspartam® | 0,10 | 0,10 |
| Acesulfam® K | 0,10 | 0,10 |
| Emulgum® (Colloides Naturels, Inc.) | 0,30 | 0,30 |
| Sorbitol, 70% | 14,00 | 14,00 |
| Glycerin | 1,00 | 1,00 |
| Minzaroma | 1,00 | - |
| Tutti-Frutti-Aroma | - | 2,00 |

FORMULIERUNGSBEISPIEL 12 Gelatinekapsel

| Zusammensetzung | 12.1 | 12.2 | 12.2 |
|---|---|---|---|
| Gelatinehülle: | | | |
| Glycerin | 2,014 | 2,014 | 2,014 |
| Gelatine 240 Bloom | 7,91 | 7,91 | 7,91 |
| Allulose | 0,065 | 0,085 | 0,10 |
| Allura-Rot | 0,006 | - | 0,011 |
| Brillantblau | 0,005 | 0,011 | - |
| Kernzusammensetzung: | | | |
| Pflanzenöl-Triglycerid (Kokosöl - Fraktion) | ad 100 | | |
| Orangen-Aroma | 10,0 | - | - |
| Pfefferminz-Aroma | - | 20,0 | - |
| Kirsch-Aroma | - | - | 28,65 |

(fortgesetzt)

| Kernzusammensetzung: | | | |
|---|---|---|---|
| Rebaudiosid A 98 % | 0,05 | 0,05 | - |
| 2-Hydroxypropylmenthylcarbonat | 0,33 | 0,20 | - |
| 2-Hydroxyethylmenthylcarbonat | - | 0,20 | 1,00 |
| (1R,3R,4S) Menthyl-3-carbonsäure-N-ethylamid (WS-3) | - | 0,55 | - |
| (-)-Menthyllactat (Frescolat ML) | - | 0,30 | - |
| Vanillin | 0,07 | - | 0,10 |

FORMULIERUNGSBEISPIEL 13 Magerjoghurt

| Zusammensetzung | 13.1 | 13.2 | 13.3 | 13.4 |
|---|---|---|---|---|
| Allulose | 8,0 | 10,0 | 8,0 | 10,0 |
| Rebaudiosid A | - | - | 0,025 | 0,025 |
| Saccharin | - | 0,3 | 0,3 | 0,3 |
| Sauerkirsch-Extrakt | 0,2 | 0,1 | 0,2 | 0,2 |
| Joghurt, 0,1 % Fett | ad 100 | | | |

FORMULIERUNGSBEISPIEL 14 Pudding

| Zusamensetzung | 14.1 | 14.2 | 14.3 |
|---|---|---|---|
| Maisstärke | 38 g | 38 g | 38 g |
| Allulose | 38 g | 28 g | 19 g |
| Zucker | - | 10 g | 19 g |
| Rebaudiosid A | - | 0,05 g | 0,08 g |
| Vanille-Aroma (Symrise) | 0,2 g | 0,2 g | 0,2 g |
| Chinolingelb | 0,02 g | 0,02 g | 0,02 g |
| Milch | 500 ml | 500 ml | 500 ml |

FORMULIERUNGSBEISPIEL 15 Brownies

| Zusammensetzung | 15.1 | 15.2 | 15.3 |
|---|---|---|---|
| Vollei | 240,0 g | 240,0 g | 240,0 g |
| Salz | 2,0 g | 2,0 g | 2,0 g |
| Wasser | - | 40,0 g | 60,0 g |
| Allulose, kristallin | 260,0 g | 260,0 g | 400,0 g |
| Allulose-Sirup, flüssig | 200,0 g | - | - |
| Allulose-Sirup, fest | - | 160,0 g | - |

(fortgesetzt)

| Zusammensetzung | 15.1 | 15.2 | 15.3 |
|---|---|---|---|
| Butter | 230,0 g | 230,0 g | 230,0 g |
| Mehl Typ 405 | 70,0 g | 70,0 g | 70,0 g |
| Kakaopulver | 110,0 g | 110,0 g | 110,0 g |
| Aroma (Vanille) | 5,0 g | 5,0 g | 5,0 g |

## Patentansprüche

1. Allulose in kristalliner Form, **dadurch gekennzeichnet, dass** sie eine Partikelgrößenverteilung bestimmt nach der $X_{c(min)}$-Methode aufweist, für die eine, zwei oder alle drei der folgenden Auswahlregeln gelten:

| Dispergierdruck [kPa] | Minimum d10 [μm] | Minimum d50 [μm] | Minimum d90 [μm] |
|---|---|---|---|
| 5 | > 70 | > 140 | > 230 |
| 20 | > 50 | > 120 | > 220 |
| 460 | > 10 | > 40 | > 130 |

und/oder
eine Partikelgrößenverteilung bestimmt nach der $X_{Fe(max)}$-Methode aufweist, für die eine, zwei oder alle drei der folgenden Auswahlregeln gelten:

| Dispergierdruck [kPa] | Minimum d10 [μm] | Minimum d50 [μm] | Minimum d90 [μm] |
|---|---|---|---|
| 5 | > 100 | > 220 | > 340 |
| 20 | > 70 | > 200 | > 330 |
| 460 | > 8 | > 65 | > 170 |

und/oder
eine Partikelgrößenverteilung bestimmt nach der $X_{area}$-Methode aufweist, für die eine, zwei oder alle drei der folgenden Auswahlregeln gelten:

| Dispergierdruck [kPa] | Minimum d10 [μm] | Minimum d50 [μm] | Minimum d90 [μm] |
|---|---|---|---|
| 5 | > 85 | > 185 | > 265 |
| 20 | > 65 | > 180 | > 260 |
| 460 | > 10 | > 50 | > 120 |

2. Allulose nach Anspruch 1, **dadurch gekennzeichnet, dass** diese einen b/l d50-Wert aufweist, für den gilt: und/oder

| Parameter | Dispergierdruck [kPa] | $X_{c(min)}$ |
|---|---|---|
| b/l d50 | 5 | 0,52-1,0 |
| und/oder | | |
| b/l d50 | 20 | 0,53-1,0 |
| und/oder | | |
| b/l d50 | 460 | 0,60 - 1,0 |

| Parameter | Dispergierdruck [kPa] | $X_{Fe(max)}$ |
|---|---|---|
| b/l d50 | 5 | 0,52-1,0 |
| und/oder | | |
| b/l d50 | 20 | 0,55-1,0 |
| und/oder | | |
| b/l d50 | 460 | 0,65-1,0 |

und/oder

| Parameter | Dispergierdruck [kPa] | $X_{area}$ |
|---|---|---|
| b/l d50 | 5 | 0,52-1,0 |
| und/oder | | |
| b/l d50 | 20 | 0,55-1,0 |
| und/oder | | |
| b/l d50 | 460 | 0,65-1,0 |

**3.** Verfahren zur Herstellung von kristalliner Allulose gemäß mindestens einem der vorstehenden Ansprüche 1 bis 2, umfassend oder bestehend aus folgenden Schritten:

(a) Bereitstellen einer wässrigen Allulose-Zubereitung, vorzugsweise einem Verdampferkonzentrat ("Allulose-Sirup"), umfassend oder bestehend aus folgenden Komponenten, bezogen auf die in der Zubereitung enthaltene Trockenmasse:

(i) etwa 90 bis etwa 99 Gew.-% Allulose,
(ii) etwa 0,5 bis etwa 10 Gew.-% Fructose,
(iii) 0 bis etwa 5 Gew.-% weitere Kohlenhydrate, verschieden von Allulose und Fructose, mit der Maßgabe, dass sich die Mengenangaben zu 100 Gew.-% ergänzen;

(b) optionale Behandlung der Allulose-Zubereitung des Schrittes (a) mit einem Entfärbungsmittel;
(c) Aufkonzentrieren der Zubereitung aus Schritt (a) oder Schritt (b) bis die Sättigungskonzentration überschritten wird;
(c) Versetzen der übersättigten Zubereitung aus Schritt (c) mit Allulose-Impfkristallen; und
(d) Abkühlen der Zubereitung aus Schritt (c) mit einer Geschwindigkeit von 0,1 bis etwa 1 K/h bis ein Kristallgehalt von etwa 30 bis etwa 50 Gew.-% erreicht wird, sowie
(e) Abtrennen und optional Trocknen der Kristalle.

**4.** Verfahren nach Anspruch 3, **dadurch gekennzeichnet, dass** die Zubereitung des Schrittes (a) einen Gehalt an Allulose ≥ 90 Gew.-%, vorzugsweise ≥ 91 Gew.-%, vorzugsweise ≥ 92 Gew.-%, vorzugsweise ≥ 93 Gew.-%, vorzugsweise ≥ 94 Gew.-%, vorzugsweise ≥ 95 Gew.-%, vorzugsweise ≥ 96 Gew.-%, vorzugsweise ≥ 97 Gew.-%, vorzugsweise ≥ 98 Gew.-%, jeweils bezogen auf die in der Zubereitung enthaltene Trockenmasse, aufweist.

**5.** Verfahren nach Anspruch 3, **dadurch gekennzeichnet, dass** die Zubereitung des Schrittes (a) einen Gehalt an Allulose ≤ 99 Gew.-%, vorzugsweise ≤ 98 Gew.-%, vorzugsweise ≤ 97 Gew.-%, vorzugsweise ≤ 96 Gew.-%, vorzugsweise ≤ 95 Gew.-%, vorzugsweise ≤ 94 Gew.-%, vorzugsweise ≤ 93 Gew.-%, vorzugsweise ≤ 92 Gew.-%, vorzugsweise ≤ 91 Gew.-%, jeweils bezogen auf die in der Zubereitung enthaltene Trockenmasse, aufweist.

**6.** Verfahren nach Anspruch 3, **dadurch gekennzeichnet, dass** die Zubereitung des Schrittes (a) einen Gehalt an Fructose von etwa 1 bis etwa 3 Gew.-% Fructose, bezogen auf die in der Zubereitung enthaltene Trockenmasse, aufweist.

**7.** Verfahren nach Anspruch 6, **dadurch gekennzeichnet, dass** die Zubereitung des Schrittes (a) einen Gehalt an

Fructose von etwa 1,5 bis etwa 2,5 Gew.-% Fructose, bezogen auf die in der Zubereitung enthaltene Trockenmasse, aufweist.

8. Verfahren nach Anspruch 3, **dadurch gekennzeichnet, dass** die Zubereitung des Schrittes (a) einen Gehalt an Fructose von etwa 0,5 bis etwa 1 Gew.-% Fructose, oder von etwa 1 bis etwa 1,5 Gew.-%, oder von etwa 1,5 bis etwa 2,0 Gew.-%, oder von etwa 2,0 bis etwa 2,5 Gew.-%, oder von etwa 2,5 bis etwa 3,0 Gew.-%, oder von etwa 3,0 bis etwa 3,5 Gew.-%, oder von etwa 3,5 bis etwa 4,0 Gew.-%, oder von etwa 4,0 bis etwa 4,5 Gew.-%, oder von etwa 4,5 bis etwa 5,0 Gew.-%, oder von etwa 5,0 bis etwa 5,5 Gew.-%, oder von etwa 5,5 bis etwa 6,0 Gew.-%, oder von etwa 6,0 bis etwa 6,5 Gew.-%, oder von etwa 6,5 bis etwa 7,0 Gew.-%, oder von etwa 7,0 bis etwa 7,5 Gew.-%, oder von etwa 7,5 bis etwa 8,0 Gew.-%, oder von etwa 8,0 bis etwa 8,5 Gew.-%, oder von etwa 8,5 bis etwa 9,0 Gew.-%, oder von etwa 9,5 bis etwa 10 Gew.-%, jeweils bezogen auf die in der Zubereitung enthaltene Trockenmasse, aufweist.

9. Verfahren nach mindestens einem der Ansprüche 3 bis 8, **dadurch gekennzeichnet, dass** man im Schritt (a) Zubereitungen einsetzt, die einen Trockensubstanzgehalt von etwa 55 bis etwa 98 Gew.-% aufweisen.

10. Verfahren nach Anspruch 9, **dadurch gekennzeichnet, dass** man im Schritt (a) Zubereitungen einsetzt, die einen Trockensubstanzgehalt von etwa 75 bis etwa 95 Gew.-% aufweisen.

11. Verfahren nach Anspruch 10, **dadurch gekennzeichnet, dass** man im Schritt (a) Zubereitungen einsetzt, die einen Feststoffgehalt von etwa 75 bis etwa 80 Gew.-%, oder von etwa 80 bis etwa 85 Gew.-%, von etwa 85 bis etwa 90 Gew.-%, von etwa 90 bis etwa 95 Gew.-%, aufweisen.

12. Verfahren nach mindestens einem der Ansprüche 3 bis 11, **dadurch gekennzeichnet, dass** die Aufkonzentrierung des Schrittes (c) bei einer Temperatur von etwa 50 bis etwa 75 °C durchgeführt wird.

13. Verfahren nach mindestens einem der Ansprüche 3 bis 12, **dadurch gekennzeichnet, dass** man der übersättigten Zubereitung Impfkristalle in Mengen von etwa 1 bis etwa 3 Gew.-% - bezogen auf die Zubereitung - zusetzt.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2018087261 A1
- WO 2016135358 A1
- WO 201820103 A1
- WO 2014186084 A1
- US 20020188019 A
- DE 102004041496
- WO 2006106023 A
- WO 2005096841 A
- WO 2006003107 A
- EP 2006067120 W
- WO 2006058893 A
- WO 2007003527 A
- US 3488419 A
- DE 2224430 A1
- DE 2343196 A1
- US 5286500 A
- WO 2002091849 A1


### In der Beschreibung aufgeführte Nicht-Patentliteratur


- **H. ITOH et al.** *Journal of Fermentation Bioengeneering*, 1995, vol. 80 (1), 101-103
- **N. WAGNER et al.** *Organic Process Research Development*, 2012, vol. 16, 323-330
- **N. WAGNER et al.** *Chemical Engineering Science*, 2015, vol. 137, 423-435
- **N. WAGNER et al.** *Angewandte Chemie Int. Ed. Engl.*, 2015, vol. 54 (14), 4182-6
- **BOSSHART et al.** *Biotechnology Bioengineering*, 2016, vol. 113 (2), 349-58
- **N. WAGNER et al.** *Journal of Chromatography A*, 2015, vol. 1398, 47-56